# EUROPEAN PATENT SPECIFICATION

(11) **EP 3 102 237 B1**
(45) Date of publication and mention of the grant of the patent: **02.12.2020**
(21) Application number: 15746743.2
(22) Date of filing: 03.02.2015
(51) Int. Cl.: A61K 31/4245, A61K 39/395, A61K 45/06, A61P 35/00

(54) **COMBINATION OF A PD-1 ANTAGONIST AND AN IDO1 INHIBITOR FOR TREATING CANCER**
KOMBINATION AUS EINEM PD-1-ANTAGONISTEN UND EINEM IDO1-INHIBITOR ZUR BEHANDLUNG VON KREBS
COMBINAISON D'UN ANTAGONISTE DE PD-1 ET D'UN INHIBITEUR DE IDO1 POUR TRAITER LE CANCER

(30) Priority: 04.02.2014 US 201461935714 P
(43) Date of publication of application: 14.12.2016
(73) Proprietor: Incyte Corporation, Wilmington, DE 19803 (US); Merck Sharp & Dohme Corp., Rahway, NJ 07065-0907 (US)
(72) Inventor: LEOPOLD, Lance, Wilmington, Delaware 19803 (US); KAUFMAN, David, North Wales, Pennsylvania 19454-1099 (US)
(74) Representative: Cornish, Kristina Victoria Joy
(86) International application number: PCT/US2015/014247
(87) International publication number: WO 2015/119944

(56) References cited:
- WO-A1-2012/135408
- WO-A1-2014/066834
- US-A1- 2010 266 617
- US-A1- 2014 023 663
- RIKKE B HOLMGAARD ET AL: "Indoleamine 2,3-dioxygenase is a critical resistance mechanism in antitumor T cell immunotherapy targeting CTLA-4", THE JOURNAL OF EXPERIMENTAL MEDICINE, ROCKEFELLER UNIVERSITY PRESS, US , vol. 210, no. 7 1 July 2013 (2013-07-01), pages 1389-1402, XP002770451, ISSN: 0022-1007 Retrieved from the Internet: URL:https://rd.springer.com/article/10.118 6/2051-1426-1-S1-P77
- STEFANI SPRANGER ET AL: "Rational combinations of immunotherapeutics that target discrete pathways", JOURNAL FOR IMMUNOTHERAPY OF CANCER, vol. 1, no. 1, 1 January 2013 (2013-01-01) , page 16, XP055237414, London, UK ISSN: 2051-1426, DOI: 10.1186/2051-1426-1-16
- TOPALIAN SUZANNE L ET AL: "Safety, activity, and immune correlates of anti-PD-1 antibody in cancer", NEW ENGLAND JOURNAL OF MEDICINE, THE - NEJM, MASSACHUSETTS MEDICAL SOCIETY, US, vol. 366, no. 26, 28 June 2012 (2012-06-28), pages 2443-2454, XP002767416, ISSN: 1533-4406
- Anonymous: "A Phase 1/2 Study Exploring the Safety, Tolerability, and Efficacy of Pembrolizumab (MK-3475) in Combination With Epacadostat (INCB024360) in Subjects With Selected Cancers (INCB 24360-202 / MK-3475-037 / KEYNOTE-037/ ECHO-202)", , 30 June 2014 (2014-06-30), XP055381569, Retrieved from the Internet: URL:https://clinicaltrials.gov/archive/NCT 02178722/2014_06_30 [retrieved on 2017-06-14]
- Anonymous: "A Study of the Safety, Tolerability, and Efficacy of Epacadostat Administered in Combination With Nivolumab in Select Advanced Cancers (ECHO-204)", , 29 December 2014 (2014-12-29), XP055381565, Retrieved from the Internet: URL:https://clinicaltrials.gov/archive/NCT 02327078/2014_12_29 [retrieved on 2017-06-14]
- PILOTTE ET AL.: 'Reversal of tumoral immune resistance by inhibition of tryptophan 2,3-dioxygenase' PNAS vol. 109, no. 107, 14 February 2012, pages 2497 - 2502, XP055219624
- DOLUSIC ET AL.: 'Indoleamine 2,3-dioxygenase inhibitors: a patent review (2008-2012' EXPERT OPIN. THER PATENTS vol. 23, no. 7, 2013, pages 1 - 15, XP009172214
- ROEHRIG ET AL.: 'Rational Design of Indoleamine 2,3-Dioxygenase Inhibitors' J. MED. CHEM. vol. 2010, no. 53, 07 January 2010, pages 1172 - 1189, XP002622031

## Description

### FIELD OF THE INVENTION

The present invention relates to combination therapies useful for the treatment of cancer. In particular, the invention relates to a combination therapy which comprises an antagonist of a Programmed Death 1 protein (PD-1) and a selective inhibitor of indoleamine 2, 3-dioxygenase 1 (IDO1).

### BACKGROUND OF THE INVENTION

PD-1 is recognized as important in immune regulation and the maintenance of peripheral tolerance. PD-1 is moderately expressed on naive T, B and NKT cells and up-regulated by T/B cell receptor signaling on lymphocytes, monocytes and myeloid cells (1).

Two known ligands for PD-1, PD-L1 (B7-H1) and PD-L2 (B7-DC), are expressed in human cancers arising in various tissues. In large sample sets of e.g. ovarian, renal, colorectal, pancreatic, liver cancers and melanoma, it was shown that PD-L1 expression correlated with poor prognosis and reduced overall survival irrespective of subsequent treatment (2-13). Similarly, PD-1 expression on tumor infiltrating lymphocytes was found to mark dysfunctional T cells in breast cancer and melanoma (14-15) and to correlate with poor prognosis in renal cancer (16). Thus, it has been proposed that PD-L1 expressing tumor cells interact with PD-1 expressing T cells to attenuate T cell activation and evasion of immune surveillance, thereby contributing to an impaired immune response against the tumor.

Several monoclonal antibodies that inhibit the interaction between PD-1 and one or both of its ligands PD-L1 and PD-L2 are in clinical development for treating cancer. It has been proposed that the efficacy of such antibodies might be enhanced if administered in combination with other approved or experimental cancer therapies, *e.g*., radiation, surgery, chemotherapeutic agents, targeted therapies, agents that inhibit other signaling pathways that are disregulated in tumors, and other immune enhancing agents.

IDO1 modulates immune cell function to a suppressive phenotype and is therefore partially accountable for tumor escape from host immune surveillance (17, 18). The enzyme indoleamine 2, 3-dioxygenase 1 (IDOl) degrades the essential amino acid tryptophan into kynurenine and other metabolites. These metabolites and the paucity of tryptophan leads to suppression of effector T-cell function and augmented differentiation of regulatory T cells (19-23). The anti-tumor efficacy of immunotherapeutic antibodies like anti-CTLA-4, anti-PD1/anti-PD-L1, and agonistic anti-GITR were significantly higher in IDO-deficient mice compared to wild type mice (24). This suggests that T-cell based immunotherapies were hampered due to IDO activity and blocking this pathway could boost the therapeutic potential of these antibodies.

INCB024360 is a selective inhibitor of IDOl enzyme activity, which is currently in clinical development by Inctye Corporation as a single agent and in combination with other modalities for multiple cancers (25, 26, 28).

MK-3475 is a selective humanized anti-human PD-1 monoclonal antibody of the IgG4/kappa isotype which is currently in clinical development by Merck as a single agent and in combination with other modalities for multiple cancers.

### SUMMARY OF THE INVENTION

The invention provides a medicament comprising an antagonist of a Programmed Death 1 protein (PD-1) for use in combination with a IDO1 inhibitor for treating a cancer in an individual, wherein the PD-1 antagonist is a monoclonal antibody with heavy chains each having the amino acid sequence of SEQ ID No: 21 and light chains each having the amino acid sequence of SEQ ID NO: 22; and the IDOl inhibitor is 4-({2-[(aminosulfonyl)amino]ethyl}amino)-N-(3-bromo-4-fluorophenyl)-N'-hydroxy-1,2,5-oxadiazole-3-carboximidamide or a pharmaceutically acceptable salt thereof.

The IDOl inhibitor of the invention may be 4-({2-[(aminosulfonyl)amino]ethyl}amino)-N-(3-bromo-4-fluorophenyl)-N'-hydroxy-1,2,5-oxadiazole-3 -carboximidamide.

The cancer to be treated by the medicament of the invention may be a solid tumor. The cancer to be treated by the medicament of the invention may be advanced renal cell carcinoma.

The PD-1 antagonist of the invention may be formulated as a liquid medicament which comprises 25 mg/ml anti-PD-1 monoclonal antibody, 7% (w/v) sucrose, 0.02% (w/v) polysorbate 80 in 10 mM histidine buffer pH 5.5.

The invention also provides a kit which comprises a first container, a second container and a package insert, wherein the first container comprises at least one dose of a medicament comprising an antagonist of a Programmed Death 1 protein (PD-1), the second container comprises at least one dose of a medicament comprising a IDO1 inhibitor, and the package insert comprises instructions for treating an individual for cancer using the medicaments, and wherein the PD-1 antagonist is a monoclonal antibody with heavy chains each having the amino acid sequence of SEQ ID NO: 21 and light chains each having the amino acid sequence of SEQ ID NO: 22, and the IDOl inhibitor is 4-({2-[(aminosulfonyl)amino]ethyl}amino)-N-(3-bromo-4-fluorophenyl)-N'-hydroxy-1,2,5-oxadiazole-3-carboximidamide or a pharmaceutically acceptable salt thereof.

The cancer to be treated by the medicaiment or kit of the present invention may be bladder cancer, breast cancer, clear cell kidney cancer, head/neck squamous cell carcinoma, lung squamous cell carcinoma, melanoma, non-small-cell lung cancer (NSCLC), ovarian cancer, pancreatic cancer, prostate cancer, renal cell cancer, small-cell lung cancer (SCLC), triple negative breast cancer, endometrial cancer, acute lymphoblastic leukemia (ALL), acute myeloid leukemia (AML), chronic lymphocytic leukemia (CLL), chronic myeloid leukemia (CML), diffuse large B-cell lymphoma (DLBCL), follicular lymphoma, Hodgkin's lymphoma (HL), mantle cell lymphoma (MCL), multiple myeloma (MM), myeloid cell leukemia-1 protein (Mcl-1), myelodysplastic syndrome (MDS), non-Hodgkin's lymphoma (NHL), or small lymphocytic lymphoma (SLL).

The PD-1 antagonist of the invention may be administered to the individual in an amount of 2 mg/kg and the IDO1 inhibitor is administered to the subject at a dose of 25 mg or 50 mg.

The PD-1 antagonist may be administered to the individual in an amount of 200 mg and the IDOl inhibitor is administered to the subject at a dose of 25 mg or 50 mg.

The IDO1 inhibitor may be administered to the individual at a dose of 25 mg BID, or wherein the IDOl inhibitor is administered to the individual at a dose of 50 mg BID.

The PD-1 antagonist may be administered every three weeks and one dose of the IDOl inhibitor is administered two times per day, preferably wherein the IDOl inhibitor is administered at twelve hour intervals.

The PD-1 antagonist and the IDOl inhibitor may be dosed over a 21-day dosing period.

The cancer to be treated by the medicament of the invention may be non-small-cell lung cancer (NSCLC), melanoma, transitional cell cancer of the bladder (TCC), renal cell cancer (RCC), or squamous cell carcinoma of the head and neck.

The invention also provides the medicament wherein
a) the PD-1 antagonist is administered to the subject in an amount of 10 mg/kg one dose every two weeks or one dose every three weeks and the IDOl inhibitor is administered to the subject at a dose of 25 mg BID, 50 mg BID, 100 mg BID, or 300 mg BID; or
b) the PD-1 antagonist is administered to the subject in an amount of 2 mg/kg one dose every two weeks or one dose every three weeks and the IDOl inhibitor is administered to the subject at a dose of 25mg BID.

There is also described use of a PD-1 antagonist in the manufacture of medicament for treating a cancer in an individual when administered in combination with an IDO1 inhibitor and use of an IDO1 inhibitor in the manufacture of a medicament for treating a cancer in an individual when administered in combination with a PD-1 antagonist.

Also described is the use of a PD-1 antagonist and an IDO1 inhibitor in the manufacture of medicaments for treating a cancer in an individual. In some preferred embodiments, the medicaments comprise a kit, and the kit also comprises a package insert comprising instructions for using the PD-1 antagonist in combination with an IDO1 inhibitor for use intreating a cancer in an individual.

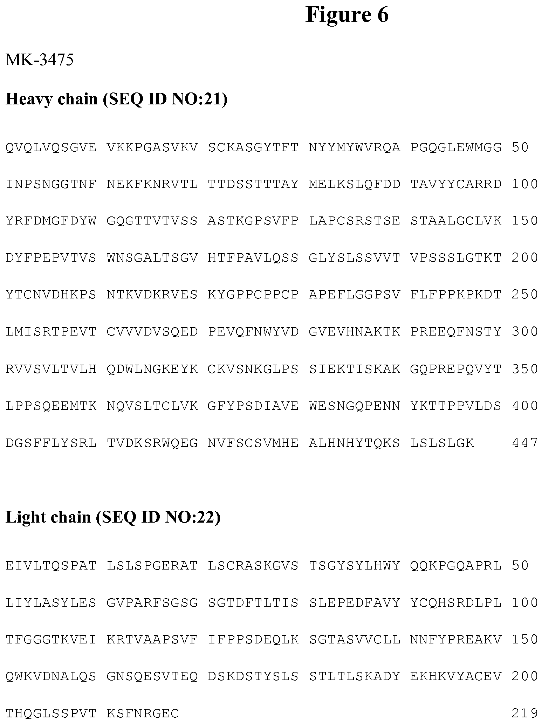

In all of the above methods, medicaments and uses, the PD-1 antagonist inhibits the binding of PD-L1 to PD-1, and preferably also inhibits the binding of PD-L2 to PD-1. In some preferred embodiments of the above methods, medicaments and uses, the PD-1 antagonist is a monoclonal antibody, or an antigen binding fragment thereof, which specifically binds to PD-1 or to PD-L1 and blocks the binding of PD-L1 to PD-1. In one particularly preferred embodiment, the PD-1 antagonist is an anti-PD-1 antibody which comprises a heavy chain and a light chain, and wherein the heavy and light chains comprise the amino acid sequences shown in Figure 6 (SEQ ID NO:21 and SEQ ID NO:22).

In all of the above embodiments of the method, medicaments and uses, the IDO1 inhibitor is a compound of Formula I: 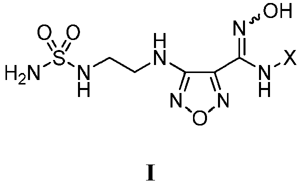 or a pharmaceutically acceptable salt thereof; wherein:
X is 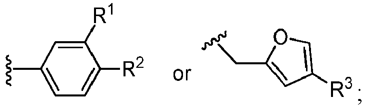
R¹ is Cl, Br, CF₃, or CN;
R² is H or F; and
R³ is Cl or Br.

In some embodiments of the above methods, medicaments and uses of the invention, the individual is a human and the cancer is a solid tumor and in some preferred embodiments, the solid tumor is transitional cell cancer of the urinary bladder, adenocarcinoma of the endometrium, bladder cancer, breast cancer, clear cell kidney cancer, head/neck squamous cell carcinoma, lung squamous cell carcinoma, melanoma, non-small-cell lung cancer (NSCLC), ovarian cancer, pancreatic cancer, prostate cancer, renal cell cancer (RCC), small-cell lung cancer (SCLC) or triple negative breast cancer. In other preferred embodiments, the cancer is advanced or metastatic NSCLC, melanoma, bladder cancer, renal cell cancer, triple negative breast cancer, endometrial cancer or squamous cell carcinoma of the head and neck, and in more preferred embodiments, the cancer is stage IIIb, stage IV or recurrent NSCLC in an individual previously treated with a platinum-based chemotherapy regimen.

In other embodiments of the above methods, medicaments and uses of the invention, the individual is a human and the cancer is a Heme malignancy and in some preferred embodiments, the Heme malignancy is acute lymphoblastic leukemia (ALL), acute myeloid leukemia (AML), chronic lymphocytic leukemia (CLL), chronic myeloid leukemia (CML), diffuse large B-cell lymphoma (DLBCL), EBV-positive DLBCL, primary mediastinal large B-cell lymphoma, T-cell/histiocyte-rich large B-cell lymphoma, follicular lymphoma, Hodgkin's lymphoma (HL), mantle cell lymphoma (MCL), multiple myeloma (MM), myeloid cell leukemia-1 protein (Mcl-1), myelodysplastic syndrome (MDS), non-Hodgkin's lymphoma (NHL), or small lymphocytic lymphoma (SLL).

Also, in preferred embodiments of any of the above methods, medicaments and uses, the cancer tests positive for the expression of one or both of PD-L1 and PD-L2. In particularly preferred embodiments, the cancer has elevated PD-L1 expression.

In one particularly preferred embodiment of the above methods, medicaments and uses, the individual is a human and the cancer is advanced or metastatic NSCLC that tests positive for human PD-L1.

### BRIEF DESCRIPTION OF THE DRAWINGS

FIGURE 1 shows amino acid sequences of the light chain and heavy chain CDRs for an exemplary anti-PD-1 monoclonal antibody useful in the present invention (SEQ ID NOs:1-6).
FIGURE 2 shows amino acid sequences of the light chain and heavy chain CDRs for another exemplary anti-PD-1 monoclonal antibody useful in the present invention (SEQ ID NOs:7-12).
FIGURE 3 shows amino acid sequences of the heavy chain variable region and full length heavy chain for an exemplary anti-PD-1 monoclonal antibody useful in the present invention (SEQ ID NO: 13 and SEQ ID NO:14).
FIGURE 4 shows amino acid sequences of alternative light chain variable regions for an exemplary anti-PD-1 monoclonal antibody useful in the present invention (SEQ ID NOs:15-17).
FIGURES 5A and 5B show amino acid sequences of alternative light chains for an exemplary anti-PD-1 monoclonal antibody useful in the present invention (SEQ ID NOs:18-20).
FIGURE 6 shows amino acid sequences of the heavy and light chains for MK-3475 (SEQ ID NOs. 21 and 22, respectively).
FIGURE 7 shows amino acid sequences of the heavy and light chains for nivolumab (SEQ ID NOs. 23 and 24, respectively).

### DETAILED DESCRIPTION

**Abbreviations.** Throughout the detailed description and examples of the invention the following abbreviations will be used:
- BID: One dose twice daily
- CDR: Complementarity determining region
- CHO: Chinese hamster ovary
- DFS: Disease free survival
- DTR: Dose limiting toxicity
- FFPE: formalin-fixed, paraffin-embedded
- FR: Framework region
- IgG: Immunoglobulin G
- IHC: Immunohistochemistry or immunohistochemical
- MTD: Maximum tolerated dose
- NCBI: National Center for Biotechnology Information
- NCI: National Cancer Institute
- OR: Overall response
- OS: Overall survival
- PD: Progressive disease
- PFS: Progression free survival
- PR: Partial response
- Q2W: One dose every two weeks
- Q3W: One dose every three weeks
- QD: One dose per day
- RECIST: Response Evaluation Criteria in Solid Tumors
- SD: Stable disease
- VH: Immunoglobulin heavy chain variable region
- VK: Immunoglobulin kappa light chain variable region

### I. DEFINITIONS

So that the invention may be more readily understood, certain technical and scientific terms are specifically defined below. Unless specifically defined elsewhere in this document, all other technical and scientific terms used herein have the meaning commonly understood by one of ordinary skill in the art to which this invention belongs.

"About" when used to modify a numerically defined parameter (*e.g*., the dose of a PD-1 antagonist or IDOl inhibitor, or the length of treatment time with a combination therapy described herein) means that the parameter may vary by as much as 10% below or above the stated numerical value for that parameter. For example, a dose of about 5 mg/kg may vary between 4.5 mg/kg and 5.5 mg/kg.

As used herein, including the appended claims, the singular forms of words such as "a," "an," and "the," include their corresponding plural references unless the context clearly dictates otherwise.

"Administration" and "treatment," as it applies to an animal, human, experimental subject, cell, tissue, organ, or biological fluid, refers to contact of an exogenous pharmaceutical, therapeutic, diagnostic agent, or composition to the animal, human, subject, cell, tissue, organ, or biological fluid. Treatment of a cell encompasses contact of a reagent to the cell, as well as contact of a reagent to a fluid, where the fluid is in contact with the cell. "Administration" and "treatment" also means *in vitro* and *ex vivo* treatments, *e.g.,* of a cell, by a reagent, diagnostic, binding compound, or by another cell. The term "subject" includes any organism, preferably an animal, more preferably a mammal (*e.g*., rat, mouse, dog, cat, rabbit) and most preferably a human.

As used herein, the term "antibody" refers to any form of antibody that exhibits the desired biological or binding activity. Thus, it is used in the broadest sense and specifically covers, but is not limited to, monoclonal antibodies (including full length monoclonal antibodies), polyclonal antibodies, multispecific antibodies (*e.g*., bispecific antibodies), humanized, fully human antibodies, chimeric antibodies and camelized single domain antibodies. "Parental antibodies" are antibodies obtained by exposure of an immune system to an antigen prior to modification of the antibodies for an intended use, such as humanization of an antibody for use as a human therapeutic.

In general, the basic antibody structural unit comprises a tetramer. Each tetramer includes two identical pairs of polypeptide chains, each pair having one "light" (about 25 kDa) and one "heavy" chain (about 50-70 kDa). The amino-terminal portion of each chain includes a variable region of about 100 to 110 or more amino acids primarily responsible for antigen recognition. The carboxy-terminal portion of the heavy chain may define a constant region primarily responsible for effector function. Typically, human light chains are classified as kappa and lambda light chains. Furthermore, human heavy chains are typically classified as mu, delta, gamma, alpha, or epsilon, and define the antibody's isotype as IgM, IgD, IgG, IgA, and IgE, respectively. Within light and heavy chains, the variable and constant regions are joined by a "J" region of about 12 or more amino acids, with the heavy chain also including a "D" region of about 10 more amino acids. See generally, Fundamental Immunology Ch. 7 (Paul, W., ed., 2nd ed. Raven Press, N.Y. (1989).

The variable regions of each light/heavy chain pair form the antibody binding site. Thus, in general, an intact antibody has two binding sites. Except in bifunctional or bispecific antibodies, the two binding sites are, in general, the same.

Typically, the variable domains of both the heavy and light chains comprise three hypervariable regions, also called complementarity determining regions (CDRs), which are located within relatively conserved framework regions (FR). The CDRs are usually aligned by the framework regions, enabling binding to a specific epitope. In general, from N-terminal to C-terminal, both light and heavy chains variable domains comprise FR1, CDR1, FR2, CDR2, FR3, CDR3 and FR4. The assignment of amino acids to each domain is, generally, in accordance with the definitions of Sequences of Proteins of Immunological Interest, Kabat, et al.; National Institutes of Health, Bethesda, Md. ; 5th ed.; NIH Publ. No. 91-3242 (1991); Kabat (1978) Adv. Prot. Chem. 32:1-75; Kabat, et al., (1977) J. Biol. Chem. 252:6609-6616; Chothia, et al., (1987) J Mol. Biol. 196:901-917 or Chothia, et al., (1989) Nature 342:878-883.

As used herein, the term "hypervariable region" refers to the amino acid residues of an antibody that are responsible for antigen-binding. The hypervariable region comprises amino acid residues from a "complementarity determining region" or "CDR" (i.e. CDRL1, CDRL2 and CDRL3 in the light chain variable domain and CDRH1, CDRH2 and CDRH3 in the heavy chain variable domain). See, Kabat et al. (1991) Sequences of Proteins of Immunological Interest, 5th Ed. Public Health Service, National Institutes of Health, Bethesda, Md. (defining the CDR regions of an antibody by sequence); see also Chothia and Lesk (1987) J. Mol. Biol. 196: 901-917 (defining the CDR regions of an antibody by structure). As used herein, the term "framework" or "FR" residues refers to those variable domain residues other than the hypervariable region residues defined herein as CDR residues.

As used herein, unless otherwise indicated, "antibody fragment" or "antigen binding fragment" refers to antigen binding fragments of antibodies, i.e. antibody fragments that retain the ability to bind specifically to the antigen bound by the full-length antibody, *e.g*. fragments that retain one or more CDR regions. Examples of antibody binding fragments include, but are not limited to, Fab, Fab', F(ab')₂, and Fv fragments; diabodies; linear antibodies; single-chain antibody molecules, *e.g*., sc-Fv; nanobodies and multispecific antibodies formed from antibody fragments.

An antibody that "specifically binds to" a specified target protein is an antibody that exhibits preferential binding to that target as compared to other proteins, but this specificity does not require absolute binding specificity. An antibody is considered "specific" for its intended target if its binding is determinative of the presence of the target protein in a sample, e.g. without producing undesired results such as false positives. Antibodies, or binding fragments thereof, useful in the present invention will bind to the target protein with an affinity that is at least two fold greater, preferably at least ten times greater, more preferably at least 20-times greater, and most preferably at least 100-times greater than the affinity with non-target proteins. As used herein, an antibody is said to bind specifically to a polypeptide *comprising* a given amino acid sequence, *e.g*. the amino acid sequence of a mature human PD-1 or human PD-L1 molecule, if it binds to polypeptides comprising that sequence but does not bind to proteins lacking that sequence.

"Chimeric antibody" refers to an antibody in which a portion of the heavy and/or light chain is identical with or homologous to corresponding sequences in an antibody derived from a particular species (*e.g*., human) or belonging to a particular antibody class or subclass, while the remainder of the chain(s) is identical with or homologous to corresponding sequences in an antibody derived from another species (*e.g*., mouse) or belonging to another antibody class or subclass, as well as fragments of such antibodies, so long as they exhibit the desired biological activity.

"Human antibody" refers to an antibody that comprises human immunoglobulin protein sequences only. A human antibody may contain murine carbohydrate chains if produced in a mouse, in a mouse cell, or in a hybridoma derived from a mouse cell. Similarly, "mouse antibody" or "rat antibody" refer to an antibody that comprises only mouse or rat immunoglobulin sequences, respectively.

"Humanized antibody" refers to forms of antibodies that contain sequences from non-human (*e.g*., murine) antibodies as well as human antibodies. Such antibodies contain minimal sequence derived from non-human immunoglobulin. In general, the humanized antibody will comprise substantially all of at least one, and typically two, variable domains, in which all or substantially all of the hypervariable loops correspond to those of a non-human immunoglobulin and all or substantially all of the FR regions are those of a human immunoglobulin sequence. The humanized antibody optionally also will comprise at least a portion of an immunoglobulin constant region (Fc), typically that of a human immunoglobulin. The prefix "hum", "hu" or "h" is added to antibody clone designations when necessary to distinguish humanized antibodies from parental rodent antibodies. The humanized forms of rodent antibodies will generally comprise the same CDR sequences of the parental rodent antibodies, although certain amino acid substitutions may be included to increase affinity, increase stability of the humanized antibody, or for other reasons.

The terms "cancer", "cancerous", or "malignant" refer to or describe the physiological condition in mammals that is typically characterized by unregulated cell growth. Examples of cancer include but are not limited to, carcinoma, lymphoma, leukemia, blastoma, and sarcoma. More particular examples of such cancers include squamous cell carcinoma, myeloma, small-cell lung cancer, non-small cell lung cancer, glioma, Hodgkin's lymphoma, non-Hodgkin's lymphoma, acute myeloid leukemia (AML), multiple myeloma, gastrointestinal (tract) cancer, renal cancer, ovarian cancer, liver cancer, lymphoblastic leukemia, lymphocytic leukemia, colorectal cancer, endometrial cancer, kidney cancer, prostate cancer, thyroid cancer, melanoma, chondrosarcoma, neuroblastoma, pancreatic cancer, glioblastoma multiforme, cervical cancer, brain cancer, stomach cancer, bladder cancer, hepatoma, breast cancer, colon carcinoma, and head and neck cancer. Particularly preferred cancers that may be treated in accordance with the present invention include those characterized by elevated expression of one or both of PD-L1 and PD-L2 in tested tissue samples.

"Biotherapeutic agent" means a biological molecule, such as an antibody or fusion protein, that blocks ligand / receptor signaling in any biological pathway that supports tumor maintenance and/or growth or suppresses the anti-tumor immune response.

"CDR" or "CDRs" as used herein means complementarity determining region(s) in a immunoglobulin variable region, defined using the Kabat numbering system, unless otherwise indicated

"Chemotherapeutic agent" is a chemical compound useful in the treatment of cancer. Classes of chemotherapeutic agents include, but are not limited to: alkylating agents, antimetabolites, kinase inhibitors, spindle poison plant alkaloids, cytotoxic/antitumor antibiotics, topisomerase inhibitors, photosensitizers, anti-estrogens and selective estrogen receptor modulators (SERMs), anti-progesterones, estrogen receptor down-regulators (ERDs), estrogen receptor antagonists, leutinizing hormone-releasing hormone agonists, anti-androgens, aromatase inhibitors, EGFR inhibitors, VEGF inhibitors, anti-sense oligonucleotides that that inhibit expression of genes implicated in abnormal cell proliferation or tumor growth. Chemotherapeutic agents useful in the treatment methods of the present invention include cytostatic and/or cytotoxic agents.

"Chothia" as used herein means an antibody numbering system described in Al-Lazikani et al., JMB 273:927-948 (1997).

"Conservatively modified variants" or "conservative substitution" refers to substitutions of amino acids in a protein with other amino acids having similar characteristics (*e.g.* charge, side-chain size, hydrophobicity/hydrophilicity, backbone conformation and rigidity, etc.), such that the changes can frequently be made without altering the biological activity or other desired property of the protein, such as antigen affinity and/or specificity. Those of skill in this art recognize that, in general, single amino acid substitutions in non-essential regions of a polypeptide do not substantially alter biological activity (*see, e.g.,* Watson et al. (1987) Molecular Biology of the Gene, The Benjamin/Cummings Pub. Co., p. 224 (4th Ed.)). In addition, substitutions of structurally or functionally similar amino acids are less likely to disrupt biological activity. Exemplary conservative substitutions are set forth in Table 1 below.

**TABLE 1. Exemplary Conservative Amino Acid Substitutions**

| **Original residue** | **Conservative substitution** |
|---|---|
| Ala (A) | Gly; Ser |
| Arg (R) | Lys; His |
| Asn (N) | Gln; His |
| Asp (D) | Glu; Asn |
| Cys (C) | Ser; Ala |
| Gln (Q) | Asn |
| Glu (E) | Asp; Gln |
| Gly (G) | Ala |
| His (H) | Asn; Gln |
| Ile (I) | Leu; Val |
| Leu (L) | Ile; Val |
| Lys (K) | Arg; His |
| Met (M) | Leu; Ile; Tyr |
| Phe (F) | Tyr; Met; Leu |
| Pro (P) | Ala |
| Ser (S) | Thr |
| Thr (T) | Ser |
| Trp (W) | Tyr; Phe |
| Tyr (Y) | Trp; Phe |
| Val (V) | Ile; Leu |

"Consists essentially of," and variations such as "consist essentially of" or "consisting essentially of," as used throughout the specification and claims, indicate the inclusion of any recited elements or group of elements, and the optional inclusion of other elements, of similar or different nature than the recited elements, that do not materially change the basic or novel properties of the specified dosage regimen, method, or composition. As a non-limiting example, a PD-1 antagonist that consists essentially of a recited amino acid sequence may also include one or more amino acids, including substitutions of one or more amino acid residues, which do not materially affect the properties of the binding compound.

"Diagnostic anti-PD-L monoclonal antibody" means a mAb which specifically binds to the mature form of the designated PD-L (PD-L1 or PDL2) that is expressed on the surface of certain mammalian cells. A mature PD-L lacks the presecretory leader sequence, also referred to as leader peptide. The terms "PD-L" and "mature PD-L" are used interchangeably herein, and shall be understood to mean the same molecule unless otherwise indicated or readily apparent from the context.

As used herein, a diagnostic anti-human PD-L1 mAb or an anti-hPD-Ll mAb refers to a monoclonal antibody that specifically binds to mature human PD-L1. A mature human PD-L1 molecule consists of amino acids 19-290 of the following sequence:

MRIFAVFIFMTYWHLLNAFTVTVPKDLYVVEYGSNMTIECKFPVEKQLDLAALIVYWEMEDKNI
IQFVHGEEDLKVQHSSYRQRARLLKDQLSLGNAALQITDVKLQDAGVYRCMISYGGADYKRITV
KVNAPYNKINQRILVVDPVTSEHELTCQAEGYPKAEVIWTSSDHQVLSGKTTTTNSKREEKLFN
VTSTLRINTTTNEIFYCTFRRLDPEENHTAELVIPELPLAHPPNERTHLVILGAILLCLGVALT
FIFRLRKGRMMDVKKCGIQDTNSKKQSDTHLEET (SEQ ID NO:25).

Specific examples of diagnostic anti-human PD-L1 mAbs useful as diagnostic mAbs for immunohistochemistry (IHC) detection of PD-L1 expression in formalin-fixed, paraffin-embedded (FFPE) tumor tissue sections are antibody 20C3 and antibody 22C3, which are described in the copending international patent application PCT/US13/075932, filed 18 December 2013. Another anti-human PD-L1 mAb that has been reported to be useful for IHC detection of PD-L1 expression in FFPE tissue sections (Chen, B.J. et al., Clin Cancer Res 19: 3462-3473 (2013)) is a rabbit anti-human PD-L1 mAb publicly available from Sino Biological, Inc. (Beijing, P.R. China; Catalog number 10084-R015).

"Framework region" or "FR" as used herein means the immunoglobulin variable regions excluding the CDR regions.

"Homology" refers to sequence similarity between two polypeptide sequences when they are optimally aligned. When a position in both of the two compared sequences is occupied by the same amino acid monomer subunit, e.g., if a position in a light chain CDR of two different Abs is occupied by alanine, then the two Abs are homologous at that position. The percent of homology is the number of homologous positions shared by the two sequences divided by the total number of positions compared × 100. For example, if 8 of 10 of the positions in two sequences are matched or homologous when the sequences are optimally aligned then the two sequences are 80% homologous. Generally, the comparison is made when two sequences are aligned to give maximum percent homology. For example, the comparison can be performed by a BLAST algorithm wherein the parameters of the algorithm are selected to give the largest match between the respective sequences over the entire length of the respective reference sequences.

The following references relate to BLAST algorithms often used for sequence analysis: BLAST ALGORITHMS: Altschul, S.F., et al., (1990) J. Mol. Biol. 215:403-410; Gish, W., et al, (1993) Nature Genet. 3:266-272; Madden, T.L., et al., (1996) Meth. Enzymol. 266:131-141; Altschul, S.F., et al., (1997) Nucleic Acids Res. 25:3389-3402; Zhang, J., et al., (1997) Genome Res. 7:649-656; Wootton, J.C., et al., (1993) Comput. Chem. 17:149-163; Hancock, J.M. et al., (1994) Comput. Appl. Biosci. 10:67-70; ALIGNMENT SCORING SYSTEMS: Dayhoff, M.O., et al., "A model of evolutionary change in proteins." in Atlas of Protein Sequence and Structure, (1978) vol. 5, suppl. 3. M.O. Dayhoff (ed.), pp. 345-352, Natl. Biomed. Res. Found., Washington, DC; Schwartz, R.M., et al, "Matrices for detecting distant relationships." in Atlas of Protein Sequence and Structure, (1978) vol. 5, suppl. 3." M.O. Dayhoff (ed.), pp. 353-358, Natl. Biomed. Res. Found., Washington, DC; Altschul, S.F., (1991) J. Mol. Biol. 219:555-565; States, D.J., et al., (1991) Methods 3:66-70; Henikoff, S., et al., (1992) Proc. Natl. Acad. Sci. USA 89:10915-10919; Altschul, S.F., et al., (1993) J. Mol. Evol. 36:290-300; ALIGNMENT STATISTICS: Karlin, S., et al., (1990) Proc. Natl. Acad. Sci. USA 87:2264-2268; Karlin, S., et al., (1993) Proc. Natl. Acad. Sci. USA 90:5873-5877; Dembo, A., et al., (1994) Ann. Prob. 22:2022-2039; and Altschul, S.F. "Evaluating the statistical significance of multiple distinct local alignments." in Theoretical and Computational Methods in Genome Research (S. Suhai, ed.), (1997) pp. 1-14, Plenum, New York.

"Isolated antibody" and "isolated antibody fragment" refers to the purification status and in such context means the named molecule is substantially free of other biological molecules such as nucleic acids, proteins, lipids, carbohydrates, or other material such as cellular debris and growth media. Generally, the term "isolated" is not intended to refer to a complete absence of such material or to an absence of water, buffers, or salts, unless they are present in amounts that substantially interfere with experimental or therapeutic use of the binding compound as described herein.

"Kabat" as used herein means an immunoglobulin alignment and numbering system pioneered by Elvin A. Kabat ((1991) Sequences of Proteins of Immunological Interest, 5th Ed. Public Health Service, National Institutes of Health, Bethesda, Md.).

"Monoclonal antibody" or "mAb" or "Mab", as used herein, refers to a population of substantially homogeneous antibodies, *i.e.,* the antibody molecules comprising the population are identical in amino acid sequence except for possible naturally occurring mutations that may be present in minor amounts. In contrast, conventional (polyclonal) antibody preparations typically include a multitude of different antibodies having different amino acid sequences in their variable domains, particularly their CDRs, which are often specific for different epitopes. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al. (1975) Nature 256: 495, or may be made by recombinant DNA methods (*see, e.g.,* U.S. Pat. No. 4,816,567). The "monoclonal antibodies" may also be isolated from phage antibody libraries using the techniques described in Clackson et al. (1991) Nature 352: 624-628 and Marks et al. (1991) J. Mol. Biol. 222: 581-597, for example. *See also* Presta (2005) J. Allergy Clin. Immunol. 116:731.

"Patient" or "subject" refers to any single subject for which therapy is desired or that is participating in a clinical trial, epidemiological study or used as a control, including humans and mammalian veterinary patients such as cattle, horses, dogs, and cats.

"PD-1 antagonist" means any chemical compound or biological molecule that blocks binding of PD-L1 expressed on a cancer cell to PD-1 expressed on an immune cell (T cell, B cell or NKT cell) and preferably also blocks binding of PD-L2 expressed on a cancer cell to the immune-cell expressed PD-1. Alternative names or synonyms for PD-1 and its ligands include: PDCD1, PD1, CD279 and SLEB2 for PD-1; PDCD1L1, PDL1, B7H1, B7-4, CD274 and B7-H for PD-L1; and PDCD1L2, PDL2, B7-DC, Btdc and CD273 for PD-L2. In any of the treatment method, medicaments and uses of the present invention in which a human individual is being treated, the PD-1 antagonist blocks binding of human PD-L1 to human PD-1, and preferably blocks binding of both human PD-L1 and PD-L2 to human PD-1. Human PD-1 amino acid sequences can be found in NCBI Locus No.: NP_005009. Human PD-L1 and PD-L2 amino acid sequences can be found in NCBI Locus No.: NP_054862 and NP_079515, respectively.

PD-1 antagonists useful in the any of the methods, medicaments and uses of the present invention include a monoclonal antibody (mAb), or antigen binding fragment thereof, which specifically binds to PD-1 or PD-L1, and preferably specifically binds to human PD-1 or human PD-L1. The mAb may be a human antibody, a humanized antibody or a chimeric antibody, and may include a human constant region. The human constant region is selected from the group consisting of IgG1, IgG2, IgG3 and IgG4 constant regions, and in preferred embodiments, the human constant region is an IgG4 constant region. In some embodiments, the antigen binding fragment is selected from the group consisting of Fab, Fab'-SH, F(ab')2, scFv and Fv fragments.

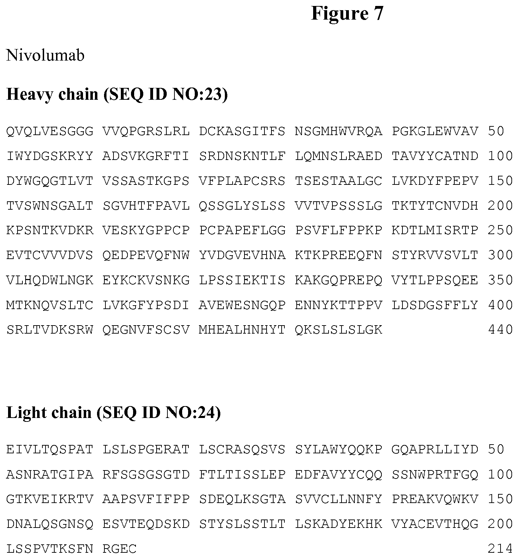

Examples of mAbs that bind to human PD-1 are described in US7488802, US7521051, US8008449, US8354509, US8168757, WO2004/004771, WO2004/072286, WO2004/056875, and US2011/0271358. The specific anti-human PD-1 mAb useful as the PD-1 antagonist in the methods, medicaments and uses of the present invention is MK-3475, a humanized IgG4 mAb with the structure described in WHO Drug Information, Vol. 27, No. 2, pages 161-162 (2013) and which comprises the heavy and light chain amino acid sequences shown in Figure 6. Another anti-human PD-1 mAb described herein is nivolumab (BMS-936558), a human IgG4 mAb with the structure described in WHO Drug Information, Vol. 27, No. 1, pages 68-69 (2013) and which comprises the heavy and light chain amino acid sequences shown in Figure 7. Disclosed herein are the humanized antibodies h409A11, h409A16 and h409A17, which are described in WO2008/156712, and AMP-514, which is being developed by MedImmune.

Examples of mAbs that bind to human PD-L1 are described in WO2013/019906, WO2010/077634 A1 and US8383796. Specific anti-human PD-L1 mAbs include MPDL3280A, BMS-936559, MEDI4736, MSB0010718C and an antibody which comprises the heavy chain and light chain variable regions of SEQ ID NO:24 and SEQ ID NO:21, respectively, of WO2013/019906.

Other PD-1 antagonists include an immunoadhesin that specifically binds to PD-1 or PD-L1, and preferably specifically binds to human PD-1 or human PD-L1, e.g., a fusion protein containing the extracellular or PD-1 binding portion of PD-L1 or PD-L2 fused to a constant region such as an Fc region of an immunoglobulin molecule. Examples of immunoadhesion molecules that specifically bind to PD-1 are described in WO2010/027827 and WO2011/066342. Specific fusion proteins include AMP-224 (also known as B7-DCIg), which is a PD-L2-FC fusion protein and binds to human PD-1.

Disclosed herein is the PD-1 antagonist as a monoclonal antibody, or antigen binding fragment thereof, which comprises: (a) light chain CDRs SEQ ID NOs: 1, 2 and 3 and heavy chain CDRs SEQ ID NOs: 4, 5 and 6; or (b) light chain CDRs SEQ ID NOs: 7, 8 and 9 and heavy chain CDRs SEQ ID NOs: 10, 11 and 12.

Disclosed herein is the PD-1 antagonist as a monoclonal antibody, or antigen binding fragment thereof, which specifically binds to human PD-1 and comprises (a) a heavy chain variable region comprising SEQ ID NO:13 or a variant thereof, and (b) a light chain variable region comprising an amino acid sequence selected from the group consisting of SEQ ID NO:15 or a variant thereof; SEQ ID NO:16 or a variant thereof; and SEQ ID NO: 17 or a variant thereof. A variant of a heavy chain variable region sequence is identical to the reference sequence except having up to 17 conservative amino acid substitutions in the framework region (i.e., outside of the CDRs), and preferably has less than ten, nine, eight, seven, six or five conservative amino acid substitutions in the framework region. A variant of a light chain variable region sequence is identical to the reference sequence except having up to five conservative amino acid substitutions in the framework region (i.e., outside of the CDRs), and preferably has less than four, three or two conservative amino acid substitution in the framework region.

Dislcosed herein is the PD-1 antagonist as a monoclonal antibody which specifically binds to human PD-1 and comprises (a) a heavy chain comprising SEQ ID NO: 14 and (b) a light chain comprising SEQ ID NO:18, SEQ ID NO:19 or SEQ ID NO:20.

Disclosed herein is the PD-1 antagonist as a monoclonal antibody which specifically binds to human PD-1 and comprises (a) a heavy chain comprising SEQ ID NO: 14 and (b) a light chain comprising SEQ ID NO:18.

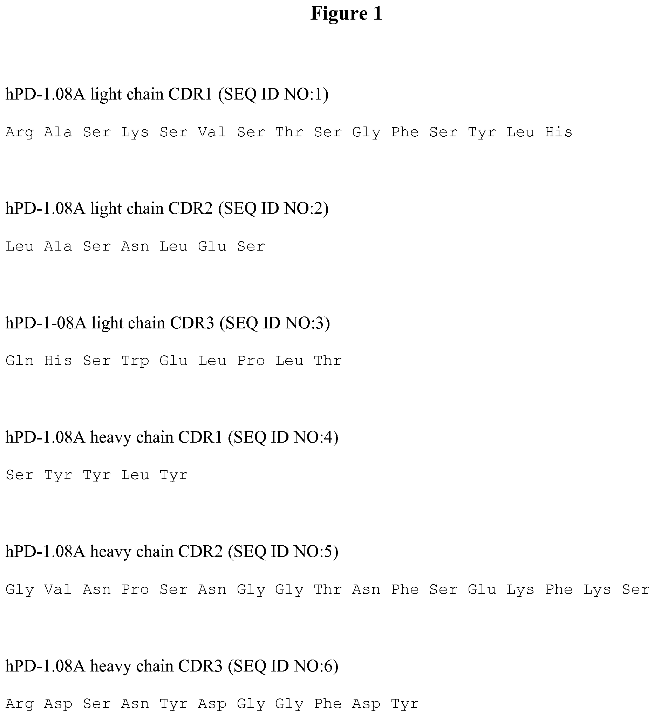

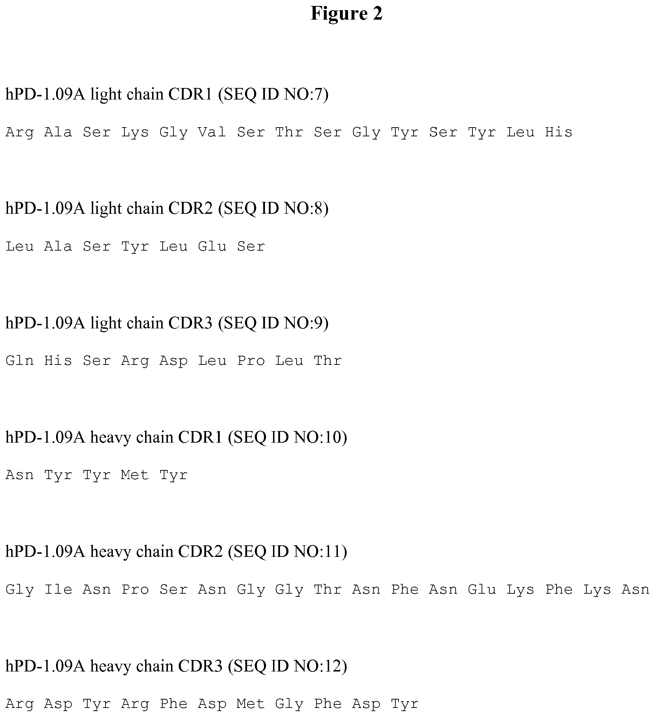

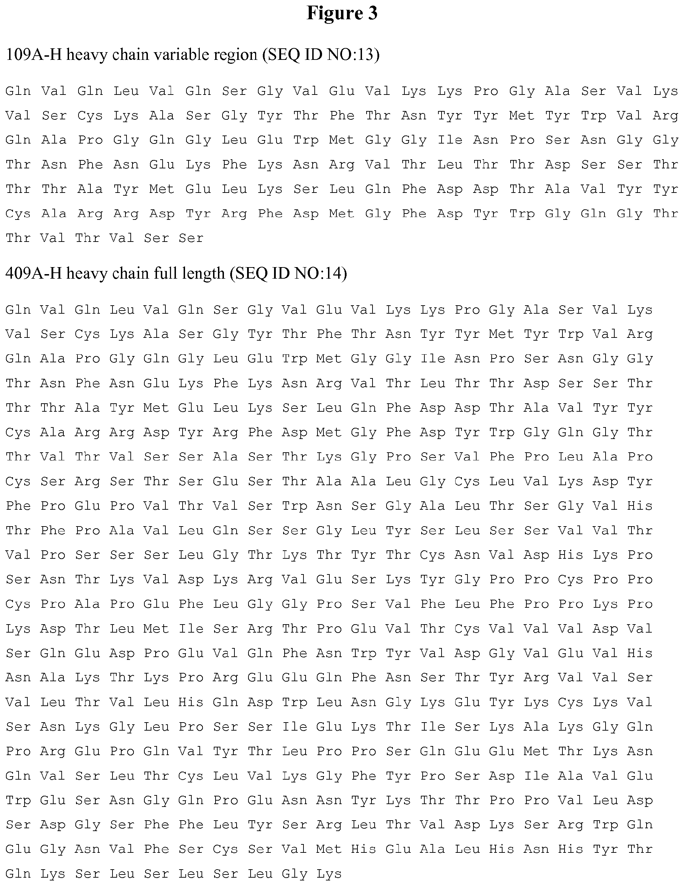

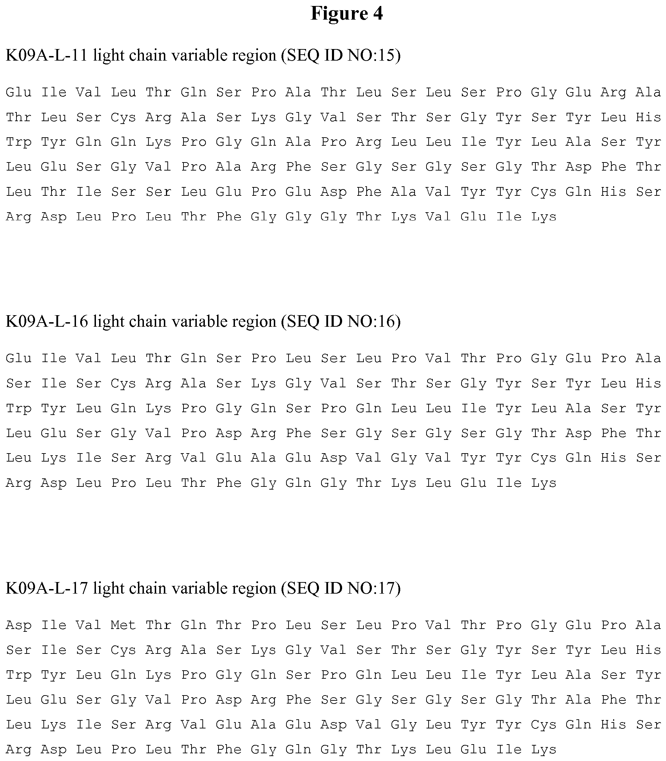

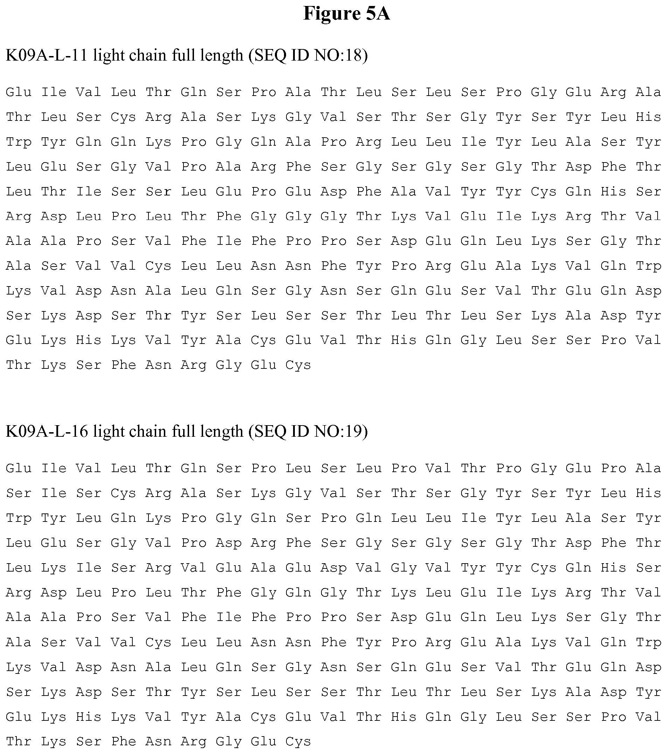

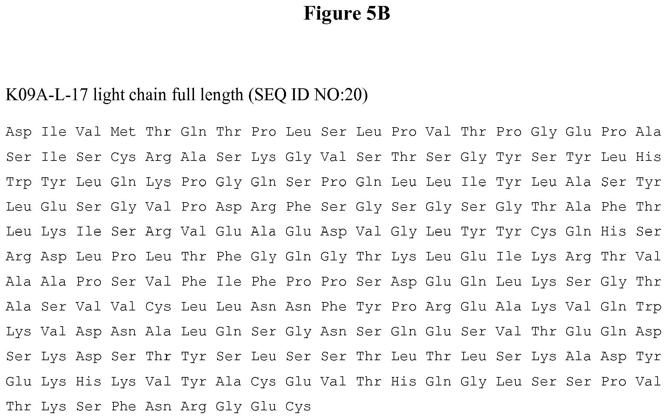

Disclosed in Table 2 below is a list of the amino acid sequences of anti-PD-1 mAbs, and the sequences are shown in Figures 1-5.

| **TABLE 2. EXEMPLARY ANTI-HUMAN PD-1 MONOCLONAL ANTIBODIES** | |
|---|---|
| **A. Comprises light and heavy chain CDRs of hPD-1.08A in WO2008/156712** | |
| CDRL1 | SEQ ID NO:1 |
| CDRL2 | SEQ ID NO:2 |
| CDRL3 | SEQ ID NO:3 |
| CDRH1 | SEQ ID NO:4 |
| CDRH2 | SEQ ID NO:5 |
| CDRH3 | SEQ ID NO:6 |

| **B. Comprises light and heavy chain CDRs of hPD-1.09A in WO2008/156712** | |
|---|---|
| CDRL1 | SEQ ID NO:7 |
| CDRL2 | SEQ ID NO:8 |
| CDRL3 | SEQ ID NO:9 |
| CDRH1 | SEQ ID NO:10 |
| CDRH2 | SEQ ID NO:11 |
| CDRH3 | SEQ ID NO:12 |

| **C. Comprises the mature h109A heavy chain variable region and one of the mature K09A light chain variable regions in WO2008/156712** | |
|---|---|
| Heavy chain VR | SEQ ID NO:13 |
| Light chain VR | SEQ ID NO:15 or SEQ ID NO:16 or SEQ ID NO:17 |

| **D. Comprises the mature 409 heavy chain and one of the mature K09A light chains in WO2008/156712** | |
|---|---|
| Heavy chain | SEQ ID NO:14 |
| Light chain | SEQ ID NO:18 or SEQ ID NO:19 or SEQ ID NO:20 |

"PD-L1" or "PD-L2" expression as used herein means any detectable level of expression of the designated PD-L protein on the cell surface or of the designated PD-L mRNA within a cell or tissue. PD-L protein expression may be detected with a diagnostic PD-L antibody in an IHC assay of a tumor tissue section or by flow cytometry. Alternatively, PD-L protein expression by tumor cells may be detected by PET imaging, using a binding agent (*e.g.,* antibody fragment, affibody and the like) that specifically binds to the desired PD-L target, *e.g.,* PD-L1 or PD-L2. Techniques for detecting and measuring PD-L mRNA expression include RT-PCR and realtime quantitative RT-PCR.

Several approaches have been described for quantifying PD-L1 protein expression in IHC assays of tumor tissue sections. *See, e.g.,* Thompson, R. H., et al., PNAS 101 (49); 17174-17179 (2004); Thompson, R. H. et al., Cancer Res. 66:3381-3385 (2006); Gadiot, J., et al., Cancer 117:2192-2201 (2011); Taube, J. M. et al., Sci Transl Med 4, 127ra37 (2012); and Toplian, S. L. et al., New Eng. J Med. 366 (26): 2443-2454 (2012).

One approach employs a simple binary end-point of positive or negative for PD-L1 expression, with a positive result defined in terms of the percentage of tumor cells that exhibit histologic evidence of cell-surface membrane staining. A tumor tissue section is counted as positive for PD-L1 expression is at least 1%, and preferably 5% of total tumor cells.

In another approach, PD-L1 expression in the tumor tissue section is quantified in the tumor cells as well as in infiltrating immune cells, which predominantly comprise lymphocytes. The percentage of tumor cells and infiltrating immune cells that exhibit membrane staining are separately quantified as < 5%, 5 to 9%, and then in 10% increments up to 100%. For tumor cells, PD-L1 expression is counted as negative if the score is < 5% score and positive if the score is ≥ 5%. PD-L1 expression in the immune infiltrate is reported as a semi-quantitative measurement called the adjusted inflammation score (AIS), which is determined by multiplying the percent of membrane staining cells by the intensity of the infiltrate, which is graded as none (0), mild (score of 1, rare lymphocytes), moderate (score of 2, focal infiltration of tumor by lymphohistiocytic aggregates), or severe (score of 3, diffuse infiltration). A tumor tissue section is counted as positive for PD-L1 expression by immune infiltrates if the AIS is ≥ 5.

The level of PD-L mRNA expression may be compared to the mRNA expression levels of one or more reference genes that are frequently used in quantitative RT-PCR, such as ubiquitin C.

In some embodiments, a level of PD-L1 expression (protein and/or mRNA) by malignant cells and/or by infiltrating immune cells within a tumor is determined to be "overexpressed" or "elevated" based on comparison with the level of PD-L1 expression (protein and/ or mRNA) by an appropriate control. For example, a control PD-L1 protein or mRNA expression level may be the level quantified in nonmalignant cells of the same type or in a section from a matched normal tissue. In some preferred embodiments, PD-L1 expression in a tumor sample is determined to be elevated if PD-L1 protein (and/or PD-L1 mRNA) in the sample is at least 10%, 20%, or 30% greater than in the control.

"RECIST 1.1 Response Criteria" as used herein means the definitions set forth in Eisenhauer *et al.,* E.A. et al., Eur. J Cancer 45:228-247 (2009) for target lesions or nontarget lesions, as appropriate based on the context in which response is being measured.

"Sustained response" (SR) means a sustained therapeutic effect after cessation of treatment with a therapeutic agent, or a combination therapy described herein. In some embodiments, the sustained response has a duration that is at least the same as the treatment duration, or at least 1.5, 2.0, 2.5 or 3 times longer than the treatment duration.

"Tissue Section" refers to a single part or piece of a tissue sample, *e.g.,* a thin slice of tissue cut from a sample of a normal tissue or of a tumor.

"Treat" or "treating" a cancer as used herein means to administer a combination therapy of a PD-1 antagonist and a IDOl inhibitor to a subject having a cancer, or diagnosed with a cancer, to achieve at least one positive therapeutic effect, such as for example, reduced number of cancer cells, reduced tumor size, reduced rate of cancer cell infiltration into peripheral organs, or reduced rate of tumor metastasis or tumor growth. Positive therapeutic effects in cancer can be measured in a number of ways (See, W. A. Weber, J. Nucl. Med. 50:1S-10S (2009)). For example, with respect to tumor growth inhibition, according to NCI standards, a T/C ≦42% is the minimum level of anti-tumor activity. A T/C < 10% is considered a high anti-tumor activity level, with T/C (%) = Median tumor volume of the treated/Median tumor volume of the control × 100. In some embodiments, the treatment achieved by a combination of the invention is any of PR, CR, OR, PFS, DFS and OS. PFS, also referred to as "Time to Tumor Progression" indicates the length of time during and after treatment that the cancer does not grow, and includes the amount of time patients have experienced a CR or PR, as well as the amount of time patients have experienced SD. DFS or "Disease Free Survival" refers to the length of time during and after treatment that the patient remains free of disease. OS refers to a prolongation in life expectancy as compared to naive or untreated individuals or patients. In some preferred embodiments, response to a combination of the invention is any of PR, CR, PFS, DFS, OR or OS that is assessed using RECIST 1.1 response criteria. In some embodiments, response to a combination of the invention is assessed using Immune Response criteria modified RECIST (irRECIST).

In some embodiments, response to a combination of the invention is assessed using Immune related response criteria (irRC).

"Bidimensional irRC" refers to the set of criteria described in Wolchok JD, et al., Guidelines for the evaluation of immune therapy activity in solid tumors: immune-related response criteria. Clin Cancer Res. 2009;15(23):7412-7420. These criteria utilize bidimensional tumor measurements of target lesions, which are obtained by multiplying the longest diameter and the longest perpendicular diameter (cm2) of each lesion.

"Unidimensional irRC refers to the set of criteria described in Nishino M, Giobbie-Hurder A, Gargano M, Suda M, Ramaiya NH, Hodi FS,. Developing a Common Language for Tumor Response to Immunotherapy: Immune-related Response Criteria using Unidimentional measurements. Clin Cancer Res. 2013;19(14):3936-3943). These criteria utilize the longest diameter (cm) of each lesion.

The treatment regimen for a combination of the invention that is effective to treat a cancer patient may vary according to factors such as the disease state, age, and weight of the patient, and the ability of the therapy to elicit an anti-cancer response in the subject. While an embodiment of any of the aspects of the invention may not be effective in achieving a positive therapeutic effect in every subject, it should do so in a statistically significant number of subjects as determined by any statistical test known in the art such as the Student's t-test, the chi²-test, the U-test according to Mann and Whitney, the Kruskal-Wallis test (H-test), Jonckheere-Terpstra-test and the Wilcoxon-test.

The terms "treatment regimen", "dosing protocol" and dosing regimen are used interchangeably to refer to the dose and timing of administration of each therapeutic agent in a combination of the invention.

"Tumor" as it applies to a subject diagnosed with, or suspected of having, a cancer refers to a malignant or potentially malignant neoplasm or tissue mass of any size, and includes primary tumors and secondary neoplasms. A solid tumor is an abnormal growth or mass of tissue that usually does not contain cysts or liquid areas. Different types of solid tumors are named for the type of cells that form them. Examples of solid tumors are sarcomas, carcinomas, and lymphomas. Leukemias (cancers of the blood) generally do not form solid tumors (National Cancer Institute, Dictionary of Cancer Terms).

"Tumor burden" also referred to as "tumor load", refers to the total amount of tumor material distributed throughout the body. Tumor burden refers to the total number of cancer cells or the total size of tumor(s), throughout the body, including lymph nodes and bone narrow. Tumor burden can be determined by a variety of methods known in the art, such as, *e.g.* by measuring the dimensions of tumor(s) upon removal from the subject, *e.g*., using calipers, or while in the body using imaging techniques, *e.g*., ultrasound, bone scan, computed tomography (CT) or magnetic resonance imaging (MRI) scans.

The term "tumor size" refers to the total size of the tumor which can be measured as the length and width of a tumor. Tumor size may be determined by a variety of methods known in the art, such as, *e.g.* by measuring the dimensions of tumor(s) upon removal from the subject, *e.g.,* using calipers, or while in the body using imaging techniques, *e.g.,* bone scan, ultrasound, CT or MRI scans.

"Variable regions" or "V region" as used herein means the segment of IgG chains which is variable in sequence between different antibodies. It extends to Kabat residue 109 in the light chain and 113 in the heavy chain.

"IDOl inhibitor" means a compound of Formula I, and pharmaceutically acceptable salts of the compound of Formula I. The IDO inhibitor includes compounds of Formula Ia and Ib, as well Compounds 1-7 *infra,* and pharmaceutically acceptable salts thereof. The compounds of Formula I may be synthesized as described in U.S. Patent No. 8,088,803, or by any other synthetic route that will be readily apparent to the skilled artisan.

In some embodiments, the IDO inhibitor is a compound of Formula I: 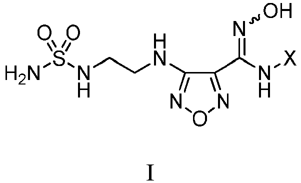 or a pharmaceutically acceptable salt thereof; wherein:
X is 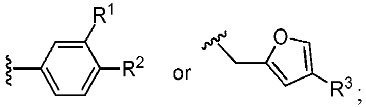
R¹ is Cl, Br, CF3, or CN;
R² is H or F; and
R³ is Cl or Br.

In some embodiments, the IDO inhibitor is a compound of Formula Ia: 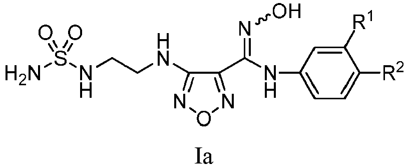 or a pharmaceutically acceptable salt thereof.

Disclosed herein is an IDO inhibitor of Formula Ib: 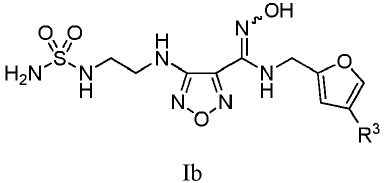 or a pharmaceutically acceptable salt thereof.

In accordance with the present invention, the IDO inhibitor is 4-({2-[(aminosulfonyl)amino]ethyl}amino)-N-(3-bromo-4-fluorophenyl)-N'-hydroxy-1,2,5-oxadiazole-3-carboximidamide (Compound 1; INCB024360): 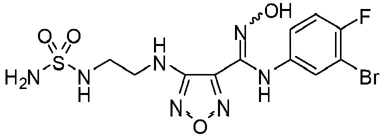 or a pharmaceutically acceptable salt thereof.

Disclosed herein is the IDO inhibitor 4-({2-[(aminosulfonyl)amino]ethyl}amino)-N-(3-chloro-4-fluorophenyl)-N'-hydroxy-1,2,5-oxadiazole-3-carboximidamide (Compound 2): 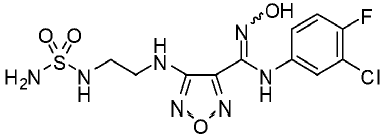 or a pharmaceutically acceptable salt thereof.

Disclosed herein is the IDO inhibitor 4-({2-[(aminosulfonyl)amino]ethyl}amino)-N-[4-fluoro-3-(trifluoromethyl)phenyl]-N'-hydroxy-1,2,5-oxadiazole-3-carboximidamide (Compound 3): 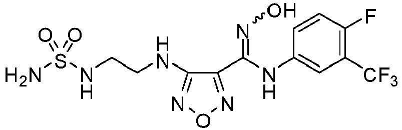 or a pharmaceutically acceptable salt thereof.

Dsiclosed herein is the IDO inhibitor 4-({2-[(aminosulfonyl)amino]ethyl}amino)-N'-hydroxy-N-[3-(trifluoromethyl)phenyl]-1,2,5-oxadiazole-3-carboximidamide (Compound 4): 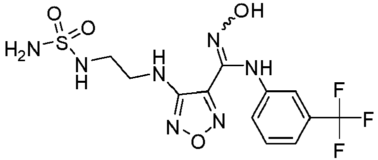 or a pharmaceutically acceptable salt thereof.

Disclosed herein is the IDO inhibitor 4-({2-[(aminosulfonyl)amino]ethyl}amino)-N-(3-cyano-4-fluorophenyl)-N'-hydroxy-1,2,5-oxadiazole-3-carboximidamide (Compound 5): 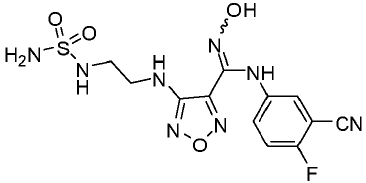 or a pharmaceutically acceptable salt thereof.

Disclosed herein is the IDO inhibitor 4-({2-[(aminosulfonyl)amino]ethyl}amino)-N-[(4-bromo-2-furyl)methyl]-N'-hydroxy-1,2,5-oxadiazole-3-carboximidamide (Compound 6): 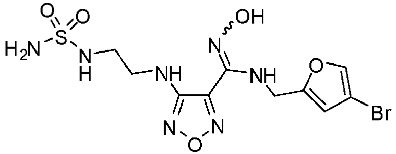 or a pharmaceutically acceptable salt thereof.

Disclosed herein is the IDO inhibitor 4-({2-[(aminosulfonyl)amino]ethyl}amino)-N-[(4-chloro-2-furyl)methyl]-N'-hydroxy-1,2,5-oxadiazole-3-carboximidamide (Compound 7): 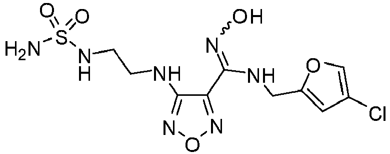 or a pharmaceutically acceptable salt thereof.

Compounds 1-7 depicted above were tested and found to be active IDO inhibitors in a human indoleamine 2,3-dioxygenasae (IDO) enzyme assay, having IC50s of <200 nM, <200 nM, <100 nM, <500 nM, <750 nM, <500 nM, and <750 nM, respectively (see U.S. Patent No. 8,088,803).

The compounds of the invention are further intended to include all possible geometric isomers. Cis and trans geometric isomers of the compounds of the present invention are described and may be isolated as a mixture of isomers or as separated isomeric forms. A bond in a structure diagram represented by a wavy line " 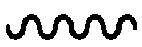 " is intended to indicate that the structure represents the cis or the trans isomer, or a mixture of the cis and trans isomers in any proportion.

Compounds of the invention also include tautomeric forms. Tautomeric forms result from the swapping of a single bond with an adjacent double bond together with the concomitant migration of a proton.

Compounds of the invention can also include all isotopes of atoms occurring in the intermediates or final compounds. Isotopes include those atoms having the same atomic number but different mass numbers. For example, isotopes of hydrogen include tritium and deuterium.

The present invention also includes salts of the compounds described herein. As used herein, "salts" refers to derivatives of the disclosed compounds wherein the parent compound is modified by converting an existing acid or base moiety to its salt form. Examples of salts include, but are not limited to, mineral acid (such as HCl, HBr, H2S04) or organic acid (such as acetic acid, benzoic acid, trifluoroacetic acid) salts of basic residues such as amines; alkali (such as Li, Na, K, Mg, Ca) or organic (such as trialkylammonium) salts of acidic residues such as carboxylic acids; and the like. The salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile (ACN) are preferred.

The "pharmaceutically acceptable salts" of the present invention include a subset of the "salts" described above which are, conventional non-toxic salts of the parent compound formed, for example, from non-toxic inorganic or organic acids. Lists of suitable salts are found in Remington's Pharmaceutical Sciences, 17th ed., Mack Publishing Company, Easton, Pa., 1985, p. 1418 and Journal of Pharmaceutical Science, 66, 2 (1977). The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

Prodrugs of the compound of Formula I are also contemplated for use in the methods, medicaments and uses of the present invention. The term "prodrug", as employed herein, denotes a compound that is a drug precursor which, upon administration to a subject, undergoes chemical conversion by metabolic or chemical processes to yield a compound of Formula I or a salt thereof. A discussion of prodrugs is provided in T. Higuchi and V. Stella, Prodrugs as Novel Delivery Systems (1987) 14 of the A.C.S. Symposium Series, and in Bioreversible Carriers in Drug Design, (1987) Edward B. Roche, ed., American Pharmaceutical Association and Pergamon Press.

### II. METHODS, USES AND MEDICAMENTS

In one aspect of the invention, the invention provides a combination therapy which comprises a PD-1 antagonist and an IDO1 inhibitor for use in treating a cancer in an individual.

The combination therapy may also comprise one or more additional therapeutic agents. The additional therapeutic agent may be, *e.g.,* a chemotherapeutic other than a VEGFR inhibitor, a biotherapeutic agent (including but not limited to antibodies to VEGF, EGFR, Her2/neu, other growth factor receptors, CD20, CD40, CD-40L, CTLA-4, OX-40, 4-1BB, and ICOS), an immunogenic agent (for example, attenuated cancerous cells, tumor antigens, antigen presenting cells such as dendritic cells pulsed with tumor derived antigen or nucleic acids, immune stimulating cytokines (for example, IL-2, IFNα2, GM-CSF), and cells transfected with genes encoding immune stimulating cytokines such as but not limited to GM-CSF).

Examples of chemotherapeutic agents include alkylating agents such as thiotepa and cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphoramide and trimethylolmelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CBI-TMI); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, fotemustine, lomustine, nimustine, ranimustine; antibiotics such as the enediyne antibiotics (*e.g*. calicheamicin, especially calicheamicin gamma1I and calicheamicin phiI1, see, *e.g*., Agnew, Chem. Intl. Ed. Engl., 33:183-186 (1994); dynemicin, including dynemicin A; bisphosphonates, such as clodronate; an esperamicin; as well as neocarzinostatin chromophore and related chromoprotein enediyne antibiotic chromomophores), aclacinomysins, actinomycin, authramycin, azaserine, bleomycins, cactinomycin, carabicin, caminomycin, carzinophilin, chromomycins, dactinomycin, daunorubicin, detorubicin, 6-diazo-5-oxo-L-norleucine, doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; lonidamine; maytansinoids such as maytansine and ansamitocins; mitoguazone; mitoxantrone; mopidamol; nitracrine; pentostatin; phenamet; pirarubicin; losoxantrone; podophyllinic acid; 2-ethylhydrazide; procarbazine; razoxane; rhizoxin; sizofuran; spirogermanium; tenuazonic acid; triaziquone; 2, 2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g. paclitaxel and doxetaxel; chlorambucil; gemcitabine; 6-thioguanine; mercaptopurine; methotrexate; platinum analogs such as cisplatin and carboplatin; vinblastine; platinum; etoposide (VP-16); ifosfamide; mitoxantrone; vincristine; vinorelbine; novantrone; teniposide; edatrexate; daunomycin; aminopterin; xeloda; ibandronate; CPT-11; topoisomerase inhibitor RFS 2000; difluoromethylornithine (DMFO); retinoids such as retinoic acid; capecitabine; and pharmaceutically acceptable salts, acids or derivatives of any of the above. Also included are anti-hormonal agents that act to regulate or inhibit hormone action on tumors such as anti-estrogens and selective estrogen receptor modulators (SERMs), including, for example, tamoxifen, raloxifene, droloxifene, 4-hydroxytamoxifen, trioxifene, keoxifene, LY117018, onapristone, and toremifene (Fareston); aromatase inhibitors that inhibit the enzyme aromatase, which regulates estrogen production in the adrenal glands, such as, for example, 4(5)-imidazoles, aminoglutethimide, megestrol acetate, exemestane, formestane, fadrozole, vorozole, letrozole, and anastrozole; and anti-androgens such as flutamide, nilutamide, bicalutamide, leuprolide, and goserelin; and pharmaceutically acceptable salts, acids or derivatives of any of the above.

Each therapeutic agent in a combination therapy of the invention may be administered either alone or in a medicament (also referred to herein as a pharmaceutical composition) which comprises the therapeutic agent and one or more pharmaceutically acceptable carriers, excipients and diluents, according to standard pharmaceutical practice.

Each therapeutic agent in a combination therapy of the invention may be administered simultaneously (i.e., in the same medicament), concurrently (i.e., in separate medicaments administered one right after the other in any order) or sequentially in any order. Sequential administration is particularly useful when the therapeutic agents in the combination therapy are in different dosage forms (one agent is a tablet or capsule and another agent is a sterile liquid) and/or are administered on different dosing schedules, *e.g*., a chemotherapeutic that is administered at least daily and a biotherapeutic that is administered less frequently, such as once weekly, once every two weeks, or once every three weeks.

In some embodiments, the IDO1 inhibitor is administered before administration of the PD-1 antagonist, while in other embodiments, the IDO1 inhibitor is administered after administration of the PD-1 antagonist.

In some embodiments, at least one of the therapeutic agents in the combination therapy is administered using the same dosage regimen (dose, frequency and duration of treatment) that is typically employed when the agent is used as monotherapy for treating the same cancer. In other embodiments, the patient receives a lower total amount of at least one of the therapeutic agents in the combination therapy than when the agent is used as monotherapy, *e.g*., smaller doses, less frequent doses, and/or shorter treatment duration.

Each small molecule therapeutic agent in a combination therapy of the invention can be administered orally or parenterally, including the intravenous, intramuscular, intraperitoneal, subcutaneous, rectal, topical, and transdermal routes of administration.

A combination therapy of the invention may be used prior to or following surgery to remove a tumor and may be used prior to, during or after radiation therapy.

In some embodiments, a combination therapy of the invention is administered to a patient who has not been previously treated with a biotherapeutic or chemotherapeutic agent, i.e., is treatment-naive. In other embodiments, the combination therapy is administered to a patient who failed to achieve a sustained response after prior therapy with a biotherapeutic or chemotherapeutic agent, i.e., is treatment-experienced.

A combination therapy of the invention is typically used to treat a tumor that is large enough to be found by palpation or by imaging techniques well known in the art, such as MRI, ultrasound, or CAT scan. In some preferred embodiments, a combination therapy of the invention is used to treat an advanced stage tumor having dimensions of at least about 200 mm³, 300 mm³, 400 mm³, 500 mm³, 750 mm³, or up to 1000 mm³.

A combination therapy of the invention is preferably administered to a human patient who has a cancer that tests positive for PD-L1 expression. In some preferred embodiments, PD-L1 expression is detected using a diagnostic anti-human PD-L1 antibody, or antigen binding fragment thereof, in an IHC assay on an FFPE or frozen tissue section of a tumor sample removed from the patient. Typically, the patient's physician would order a diagnostic test to determine PD-L1 expression in a tumor tissue sample removed from the patient prior to initiation of treatment with the PD-1 antagonist and IDOl inhibitor, but it is envisioned that the physician could order the first or subsequent diagnostic tests at any time after initiation of treatment, such as for example after completion of a treatment cycle.

Selecting a dosage regimen (also referred to herein as an administration regimen) for a combination therapy of the invention depends on several factors, including the serum or tissue turnover rate of the entity, the level of symptoms, the immunogenicity of the entity, and the accessibility of the target cells, tissue or organ in the individual being treated. Preferably, a dosage regimen maximizes the amount of each therapeutic agent delivered to the patient consistent with an acceptable level of side effects. Accordingly, the dose amount and dosing frequency of each biotherapeutic and chemotherapeutic agent in the combination depends in part on the particular therapeutic agent, the severity of the cancer being treated, and patient characteristics. Guidance in selecting appropriate doses of antibodies, cytokines, and small molecules are available. *See, e.g.,* Wawrzynczak (1996) Antibody Therapy, Bios Scientific Pub. Ltd, Oxfordshire, UK; Kresina (ed.) (1991) Monoclonal Antibodies, Cytokines and Arthritis, Marcel Dekker, New York, NY; Bach (ed.) (1993) Monoclonal Antibodies and Peptide Therapy in Autoimmune Diseases, Marcel Dekker, New York, NY; Baert et al. (2003) New Engl. J. Med. 348:601-608; Milgrom et al. (1999) New Engl. J. Med. 341:1966-1973; Slamon et al. (2001) New Engl. J. Med. 344:783-792; Beniaminovitz et al. (2000) New Engl. J. Med. 342:613-619; Ghosh et al. (2003) New Engl. J. Med. 348:24-32; Lipsky et al. (2000) New Engl. J. Med. 343:1594-1602; Physicians' Desk Reference 2003 (Physicians' Desk Reference, 57th Ed); Medical Economics Company; ISBN: 1563634457; 57th edition (November 2002). Determination of the appropriate dosage regimen may be made by the clinician, *e.g*., using parameters or factors known or suspected in the art to affect treatment or predicted to affect treatment, and will depend, for example, the patient's clinical history (*e.g*., previous therapy), the type and stage of the cancer to be treated and biomarkers of response to one or more of the therapeutic agents in the combination therapy.

Biotherapeutic agents in a combination therapy of the invention may be administered by continuous infusion, or by doses at intervals of, *e.g.,* daily, every other day, three times per week, or one time each week, two weeks, three weeks, monthly, bimonthly, etc. A total weekly dose is generally at least 0.05 µg/kg, 0.2 µg/kg, 0.5 µg/kg, 1 µg/kg, 10 µg/kg, 100 µg/kg, 0.2 mg/kg, 1.0 mg/kg, 2.0 mg/kg, 10 mg/kg, 25 mg/kg, 50 mg/kg body weight or more. *See, e.g.,* Yang et al. (2003) New Engl. J. Med. 349:427-434; Herold et al. (2002) New Engl. J. Med. 346:1692-1698; Liu et al. (1999) J. Neurol. Neurosurg. Psych. 67:451-456; Portielji et al. (20003) Cancer Immunol. Immunother. 52:133-144.

In some embodiments that employ an anti-human PD-1 mAb as the PD-1 antagonist in the combination therapy, the dosing regimen will comprise administering the anti-human PD-1 mAb at a dose of 1, 2, 3, 5 or 10mg/kg at intervals of about 14 days (± 2 days) or about 21 days (± 2 days) or about 30 days (± 2 days) throughout the course of treatment.

In other embodiments that employ an anti-human PD-1 mAb as the PD-1 antagonist in the combination therapy, the dosing regimen will comprise administering the anti-human PD-1 mAb at a dose of from about 0.005 mg/kg to about 10 mg/kg, with intra-patient dose escalation. In other escalating dose embodiments, the interval between doses will be progressively shortened, *e.g*., about 30 days (± 2 days) between the first and second dose, about 14 days (± 2 days) between the second and third doses. In certain embodiments, the dosing interval will be about 14 days (± 2 days), for doses subsequent to the second dose.

In certain embodiments, a subject will be administered an intravenous (IV) infusion of a medicament comprising any of the PD-1 antagonists described herein.

Disclosed herein is the PD-1 antagonist in the combination therapy as nivolumab, which is administered intravenously at a dose selected from the group consisting of: 1 mg/kg Q2W, 2 mg/kg Q2W, 3 mg/kg Q2W, 5 mg/kg Q2W, 10 mg Q2W, 1 mg/kg Q3W, 2 mg/kg Q3W, 3 mg/kg Q3W, 5 mg/kg Q3W, and 10 mg Q3W.

In accordance with the present invention, the PD-1 antagonist in the combination therapy is MK-3475, which is administered in a liquid medicament at a dose selected from the group consisting of 1 mg/kg Q2W, 2 mg/kg Q2W, 3 mg/kg Q2W, 5 mg/kg Q2W, 10 mg Q2W, 1 mg/kg Q3W, 2 mg/kg Q3W, 3 mg/kg Q3W, 5 mg/kg Q3W, 10 mg Q3W and flat-dose equivalents of any of these doses, such as 200 mg Q3W. In some particularly preferred embodiments, MK-3475 is administered as a liquid medicament which comprises 25 mg/ml MK-3475, 7% (w/v) sucrose, 0.02% (w/v) polysorbate 80 in 10 mM histidine buffer pH 5.5, and the selected dose of the medicament is administered by IV infusion over a time period of about 30 minutes.

The optimal dose for a compound of Formula I in combination with MK-3475 may be identified by dose escalation of one or both of these agents. In one embodiment, MK-3475 is administered at a starting dose of 10 mg/kg Q2W or Q3W and the compound of Formula I (*e.g*., INCB024360) is administered at a starting dose of 25 mg BID, 50 mg BID, 100 mg BID, or 300 mg BID. If the starting dose combination is not tolerated by the patient, then the dose of MK-3475 is reduced to 2 mg/kg Q2W or 2 mg/kg Q3W and the compound of Formula I (e*.g.,* INCB024360) is administered at 25 mg BID.

In an embodiment, 25 mg of the compound of Formula I (e.g., INCB024360) is administered with or without food BID, with each dose administered about 12 hours apart. On the day of MK-3475 administration in a treatment cycle, the compound of Formula I (*e.g.,* INCB024360) may be given prior to or after the MK-3475 administration.

In some embodiments, the patient is treated with each agent sequentially, and in one preferred embodiment, the compound of Formula I (*e.g*., INCB024360) at 25 mg is administered for a treatment cycle of ten days followed by MK-3475 once every five days (*e.g*., two cycles). In another preferred embodiment, 25 mg MK-3475 is administered once every five days for 2 cycles followed by the compound of Formula I (*e.g*., INCB024360).

In a particular embodiment directed to a combination of the invention, the compound of Formula I (*e.g*., INCB024360) is orally administered BID at a dose of 25 mg or 50 mg during a 21 day cycle for an every three week (Q3W) dose schedule of MK-3475. In a particular embodiment, the compound of Formula I (*e.g*., INCB024360) is orally administered BID approximately 12 hours apart. In a particular embodiment, MK-3475 is administered at a dose of 2 mg/kg or 200 mg IV over a 30-minute period on Day 1 of each 3-week cycle and the compound of Formula I (*e.g*., INCB024360) is orally administered BID at a dose of 25 mg or 50 mg. In an embodiment, the BID dose of compound of Formula I (*e.g*., INCB024360) is administered 12 hours apart, without respect to food. In an embodiment, the compound of Formula I (*e.g*., INCB024360) is orally administered BID at a dose of 25 mg. In another embodiment, the compound of Formula I (*e.g*., INCB024360) is orally administered BID at a dose of 50 mg. In an embodiment, the compound of Formula I (*e.g*., INCB024360) is orally administered BID at a dose of 25 mg in combination with a dose of MK-3475 of 2 mg/kg IV In an embodiment the compound of Formula I (*e.g*., INCB024360) is orally administered BID at a dose of 25 mg in combination with a dose of MK-3475 of 200 mg IV In an embodiment the compound of Formula I (*e.g*., INCB024360) is orally administered BID at a dose of 50 mg in combination with a dose of MK-3475 of 2 mg/kg IV In an embodiment the compound of Formula I (*e.g*., INCB024360) is orally administered BID at a dose of 50 mg in combination with a dose of MK-3475 of 200 mg IV Specific embodiments include, for example, MK-3475 dosed at 2 mg/kg every three weeks with either 25 mg or 50 mg of INCB024360 BID; and MK-3475 dosed at 200 mg every three weeks with either 25 mg or 50 mg of INCB024360 BID. Treatment cycles may be continued for as long as patients receive benefit from the treatment. In some embodiments, treatment is continued for 3, 6, 9, 12, 15, 18, or 24 months.

In some embodiments, a treatment cycle begins with the first day of combination treatment and lasts for 2 weeks or 3 weeks. In such embodiments, the combination therapy is preferably administered for at least 12 weeks (6 cycles of treatment), more preferably at least 24 weeks, 36 weeks or 48 weeks, and even more preferably at least 2 weeks after the patient achieves a CR.

In some embodiments, the patient who is selected for treatment with the combination therapy of the invention has NSCLC which tests positive for elevated PD-L1 expression.

The present invention also provides a medicament which comprises a PD-1 antagonist as described above and a pharmaceutically acceptable excipient. When the PD-1 antagonist is a biotherapeutic agent, *e.g.,* a mAb, the antagonist may be produced in CHO cells using conventional cell culture and recovery/purification technologies.

In some embodiments, a medicament comprising an anti-PD-1 antibody as the PD-1 antagonist may be provided as a liquid formulation or prepared by reconstituting a lyophilized powder with sterile water for injection prior to use. WO 2012/135408 describes the preparation of liquid and lyophilized medicaments comprising MK-3475 that are suitable for use in the present invention. In some preferred embodiments, a medicament comprising MK-3475 is provided in a glass vial which contains about 50 mg of MK-3475.

The present invention also provides a medicament which comprises a compound of Formula I and a pharmaceutically acceptable excipient. The compound of Formula I may be prepared as described in U.S. Patent No. 8,088,803, and may be formulated as described therein. In one embodiment, the compound of Formula I is INCB024360.

The anti-PD-1 and compound of Formula I medicaments described herein may be provided as a kit which comprises a first container and a second container and a package insert. The first container contains at least one dose of a medicament comprising an anti-PD-1 antagonist, the second container contains at least one dose of a medicament comprising a compound of Formula I (*e.g*., INCB024360), and the package insert, or label, which comprises instructions for treating a patient for cancer using the medicaments. The first and second containers may be comprised of the same or different shape (*e.g*., vials, syringes and bottles) and/or material (*e.g*., plastic or glass). The kit may further comprise other materials that may be useful in administering the medicaments, such as diluents, filters, IV bags and lines, needles and syringes. In some preferred embodiments of the kit, the anti-PD-1 antagonist is an anti-PD-1 antibody and the instructions state that the medicaments are intended for use in treating a patient having a cancer that tests positive for PD-L1 expression by an IHC assay.

These and other aspects of the invention, including the exemplary specific embodiments listed below, will be apparent from the teachings contained herein.

### GENERAL METHODS

Standard methods in molecular biology are described Sambrook, Fritsch and Maniatis (1982 & 1989 2nd Edition, 2001 3rd Edition) Molecular Cloning, A Laboratory Manual, Cold Spring Harbor Laboratory Press, Cold Spring Harbor, NY; Sambrook and Russell (2001) Molecular Cloning, 3rd ed., Cold Spring Harbor Laboratory Press, Cold Spring Harbor, NY; Wu (1993) Recombinant DNA, Vol. 217, Academic Press, San Diego, CA). Standard methods also appear in Ausbel, et al. (2001) Current Protocols in Molecular Biology, Vols. 1-4, John Wiley and Sons, Inc. New York, NY, which describes cloning in bacterial cells and DNA mutagenesis (Vol. 1), cloning in mammalian cells and yeast (Vol. 2), glycoconjugates and protein expression (Vol. 3), and bioinformatics (Vol. 4).

Methods for protein purification including immunoprecipitation, chromatography, electrophoresis, centrifugation, and crystallization are described (Coligan, et al. (2000) Current Protocols in Protein Science, Vol. 1, John Wiley and Sons, Inc., New York). Chemical analysis, chemical modification, post-translational modification, production of fusion proteins, glycosylation of proteins are described (see, *e.g.,* Coligan, et al. (2000) Current Protocols in Protein Science, Vol. 2, John Wiley and Sons, Inc., New York; Ausubel, et al. (2001) Current Protocols in Molecular Biology, Vol. 3, John Wiley and Sons, Inc., NY, NY, pp. 16.0.5-16.22.17; Sigma-Aldrich, Co. (2001) Products for Life Science Research, St. Louis, MO; pp. 45-89; Amersham Pharmacia Biotech (2001) BioDirectory, Piscataway, N.J., pp. 384-391). Production, purification, and fragmentation of polyclonal and monoclonal antibodies are described (Coligan, et al. (2001) Current Protcols in Immunology, Vol. 1, John Wiley and Sons, Inc., New York; Harlow and Lane (1999) Using Antibodies, Cold Spring Harbor Laboratory Press, Cold Spring Harbor, NY; Harlow and Lane, *supra*). Standard techniques for characterizing ligand/receptor interactions are available (see, *e.g.,* Coligan, et al. (2001) Current Protocols in Immunology, Vol. 4, John Wiley, Inc., New York).

Monoclonal, polyclonal, and humanized antibodies can be prepared (see, *e.g.,* Sheperd and Dean (eds.) (2000) Monoclonal Antibodies, Oxford Univ. Press, New York, NY; Kontermann and Dubel (eds.) (2001) Antibody Engineering, Springer-Verlag, New York; Harlow and Lane (1988) Antibodies A Laboratory Manual, Cold Spring Harbor Laboratory Press, Cold Spring Harbor, NY, pp. 139-243; Carpenter, et al. (2000) J. Immunol. 165:6205; He, et al. (1998) J. Immunol. 160:1029; Tang et al. (1999) J. Biol. Chem. 274:27371-27378; Baca et al. (1997) J. Biol. Chem. 272:10678-10684; Chothia et al. (1989) Nature 342:877-883; Foote and Winter (1992) J. Mol. Biol. 224:487-499; U.S. Pat. No. 6,329,511).

An alternative to humanization is to use human antibody libraries displayed on phage or human antibody libraries in transgenic mice (Vaughan et al. (1996) Nature Biotechnol. 14:309-314; Barbas (1995) Nature Medicine 1:837-839; Mendez et al. (1997) Nature Genetics 15:146-156; Hoogenboom and Chames (2000) Immunol. Today 21:371-377; Barbas et al. (2001) Phage Display: A Laboratory Manual, Cold Spring Harbor Laboratory Press, Cold Spring Harbor, New York; Kay et al. (1996) Phage Display of Peptides and Proteins: A Laboratory Manual, Academic Press, San Diego, CA; de Bruin et al. (1999) Nature Biotechnol. 17:397-399).

Purification of antigen is not necessary for the generation of antibodies. Animals can be immunized with cells bearing the antigen of interest. Splenocytes can then be isolated from the immunized animals, and the splenocytes can fused with a myeloma cell line to produce a hybridoma (see, *e.g.,* Meyaard et al. (1997) Immunity 7:283-290; Wright et al. (2000) Immunity 13:233-242; Preston *et al., supra;* Kaithamana et al. (1999) J. Immunol. 163:5157-5164).

Antibodies can be conjugated, *e.g*., to small drug molecules, enzymes, liposomes, polyethylene glycol (PEG). Antibodies are useful for therapeutic, diagnostic, kit or other purposes, and include antibodies coupled, *e.g.,* to dyes, radioisotopes, enzymes, or metals, *e.g.,* colloidal gold (see, *e.g.,* Le Doussal et al. (1991) J. Immunol. 146:169-175; Gibellini et al. (1998) J. Immunol. 160:3891-3898; Hsing and Bishop (1999) J. Immunol. 162:2804-2811; Everts et al. (2002) J. Immunol. 168:883-889).

Methods for flow cytometry, including fluorescence activated cell sorting (FACS), are available (see, *e.g.,* Owens, et al. (1994) Flow Cytometry Principles for Clinical Laboratory Practice, John Wiley and Sons, Hoboken, NJ; Givan (2001) Flow Cytometry, 2nd ed.; Wiley-Liss, Hoboken, NJ; Shapiro (2003) Practical Flow Cytometry, John Wiley and Sons, Hoboken, NJ). Fluorescent reagents suitable for modifying nucleic acids, including nucleic acid primers and probes, polypeptides, and antibodies, for use, *e.g.,* as diagnostic reagents, are available (Molecular Probesy (2003) Catalogue, Molecular Probes, Inc., Eugene, OR; Sigma-Aldrich (2003) Catalogue, St. Louis, MO).

Standard methods of histology of the immune system are described (see, *e.g.,* Muller-Harmelink (ed.) (1986) Human Thymus: Histopathology and Pathology, Springer Verlag, New York, NY; Hiatt, et al. (2000) Color Atlas of Histology, Lippincott, Williams, and Wilkins, Phila, PA; Louis, et al. (2002) Basic Histology: Text and Atlas, McGraw-Hill, New York, NY).

Software packages and databases for determining, *e.g*., antigenic fragments, leader sequences, protein folding, functional domains, glycosylation sites, and sequence alignments, are available (see, *e.g*., GenBank, Vector NTI® Suite (Informax, Inc, Bethesda, MD); GCG Wisconsin Package (Accelrys, Inc., San Diego, CA); DeCypher® (TimeLogic Corp., Crystal Bay, Nevada); Menne, et al. (2000) Bioinformatics 16: 741-742; Menne, et al. (2000) Bioinformatics Applications Note 16:741-742; Wren, et al. (2002) Comput. Methods Programs Biomed. 68:177-181; von Heijne (1983) Eur. J. Biochem. 133:17-21; von Heijne (1986) Nucleic Acids Res. 14:4683-4690).

### EXAMPLE 1

### Clinical study evaluating a combination of the invention in the treatment of patients with solid tumors

This Example describes an ongoing Phase 1/2 clinical study to evaluate the safety, tolerability and efficacy of the inventive combination of MK-3475 and INCB024360 in subjects presenting with various solid tumors and advanced non-small cell lung cancer (NSCLC). Phase 1 of the study is a dose-escalation phase and Phase 2 is randomized, double-blind, and placebo-controlled. Phase 1 of the study is conducted in subjects with selected advanced or metastatic solid tumors. Phase 2 of the study is conducted in subjects with advanced NSCLC, e.g., Stage IIIB, IV, or recurrent NSCLC. The dose-escalation phase (Phase 1) is open-label and is designed to identify the maximum tolerated dose (MTD) or pharmacologically acceptable doses (PAD) of INCB024360 in combination with MK-3475 in subjects with Stage IIIB, IV, or recurrent NSCLC, melanoma, transitional cell carcinoma of the genitourinary (GU) tract, renal cell cancer, triple negative breast cancer, adenocarcinoma of the endometrium or squamous cell carcinoma of the head and neck who have disease progression on at least 1 line of therapy for advanced or metastatic cancer.

Phase 2 further explores the safety and efficacy of the recommended dose of INCB024360 in combination with MK-3475 determined in Phase 1 in subjects with NSCLC whose disease has progressed on 1 platinum-based chemotherapy regimen for Stage IIIB, IV, or recurrent NSCLC. In the Phase 2 randomized portion of the study, subjects are randomized to receive MK-3475 with either INCB024360 or placebo and are stratified by PD-L1 expression (high vs negative/low). Phase 2 randomization is 1:1 (active: placebo).

The Phase 1 dose escalation portion of the study involves approximately 45 subjects, followed by approximately 82 subjects in the Phase 2 randomized portion of the study (randomized 1:1 in each of 2 treatment groups).

The Phase 1 dose-escalation phase includes cohorts of subjects treated with INCB024360 twice daily (BID) at initial doses of 25 mg BID, 50 mg BID, and 100 mg BID in combination with MK-3475 at 2 mg/kg every 3 weeks. One treatment cycle consists of 21 days. A minimum of 3 subjects are enrolled and treated in each cohort, and all 3 subjects are observed for a minimum of 42 days (6 weeks) before the subsequent cohort begins enrollment. Additional subjects are enrolled in a cohort to achieve the minimum of 3 evaluable subjects if dropouts or dose interruptions or reductions occur that result in a subject being nonevaluable for dose limiting toxicities (DLTs). When the MTD or PAD is reached, additional subjects are enrolled for a total of 9 subjects, but the additional subjects are treated with MK-3475 at 200 mg every 3 weeks and the MTD or PAD of INCB024360 to further evaluate safety and confirm it as the recommended Phase 2 dose (RP2D). The study subject cohorts are summarized as follows:

| **Cohort** | **Daily Dose of INCB024360** | **Dose of MK-3475 (Q3W)** |
|---|---|---|
| 1 | 25 mg BID orally | 2 mg/kg IV |
| 2 | 50 mg BID orally | 2 mg/kg IV |

Based on the results of the study cohorts 1 and 2, dose escalations of INCB024360 may also be evaluated, such as 100 mg or 300 mg BID orally in combination with 2 mg/kg MK-3475 IV Q3W.

For Phase 2 randomization, 82 subjects are randomized 1:1 into 2 treatment groups: Group 1 cohort subjects receive MK-3475 200 mg every 3 weeks + INCB024360 (41 subjects); Group 2 cohort subjects receive MK-3475 200 mg every 3 weeks + matching placebo (41 subjects). Subjects are stratefied by PD-L1 expression (high versus negative/low).

In accordance with the Phase 1/2 study, screening is up to 28 days. The treatment period with the combination therapy (MK-3475 + INCB024360 or placebo) continues every 21 days for up to 24 months, and then treatment with monotherapy INCB024360 may continue as long as subjects are receiving benefit from treatment and have not had disease progression or met any criteria for study withdrawal. Subjects who complete 24 months of MK-3475, continue on monotherapy INCB024360, and later experience disease progression on INCB024360 monotherapy may be considered for re-treatment with the combination for an additional 12 months followed by monotherapy INCB024360 as long as they are receiving benefit from treatment and have not met any criteria for study withdrawal. Safety follow-up visits occur 42-49 days after the last dose of INCB024360 is taken. For follow-up, blood samples are collected from all subjects for pharmacokinetics (PK) and antidrug antibody (ADA) at 1 month, 3 months and 6 months after the last dose of MK-3475. Subjects who discontinue for reasons other than pharmacodynamic or progressive disease (PD) have post-treatment follow-up for disease status until disease progression, initiating a nonstudy cancer treatment, withdrawing consent, or becoming lost to follow-up. For survival follow-up, all subjects are followed every 12 weeks from the last dose of treatment. Tumor assessments may also continue for subjects who are withdrawn from the study for reasons other than disease progression every 9 weeks for the first 18 months then every 12 weeks until a new cancer therapy is started, disease progression, or death.

For treatment, INCB024360 is self-administered orally BID and continued BID during the 21-day cycle for an every-3-week dose schedule of MK-3475. The MTD of INCB024360 (or PAD) defined during Phase 1 is used for Phase 2. All BID doses are taken morning and evening, approximately 12 hours apart without respect to food. If a dose is missed by more than 4 hours, that dose is skipped and is resumed at the scheduled time. MK-3475 is administered at 2 mg/kg or 200 mg intravenously (IV) over a 30-minute period on Day 1 of an every-3-week cycle, with no intrasubject dose escalation. Study subject participation averages about 6 months.

The Phase 1 study subjects are those with Stage IIIB, IV, or recurrent NSCLC, melanoma, transitional cell carcinoma of the GU tract, renal cell cancer, triple negative breast cancer, adenocarcinoma of the endometrium, or squamous cell carcinoma of the head and neck who have received at least 1 line of prior therapy and are refractory or for which no curative treatment is available will be enrolled. The Phase 2 study subjects are those with Stage IIIB, IV, or recurrent NSCLC who had disease progression on or were intolerant to platinum-based therapy or who had disease progression within 6 months of completing adjuvant therapy that included platinum-based therapy will be enrolled.

The key inclusion criteria of the Phase 1/2 study are as follows: male or female subjects, age 18 years or older; willingness to provide written informed consent/assent for the study; histologically or cytologically confirmed NSCLC, melanoma, transitional cell carcinoma of the GU tract, renal cell cancer, triple negative breast cancer, adenocarcinoma of the endometrium, or squamous cell carcinoma of the head and neck (Phase 1) or Stage IIIB, IV, or recurrent NSCLC (Phase 2); life expectancy > 12 weeks; Eastern Cooperative Oncology Group (ECOG) performance status 0 to 1; presence of measurable disease per RECIST v1.1; laboratory and medical history parameters within the protocol-defined ranges. For the Phase 1 study, inclusion criteria include subjects who have advanced or metatstatic disease who have received at least 1 prior therapy for their disease under study or who have advanced or metatstatic disease for which no curative treatment is available. For the Phase 2 study,

For the Phase 2 study, subjects are included who have received only 1 prior systemic chemotherapy regimen for Stage IIIB, IV, or recurrent NSCLC (not including neoadjuvant and/or adjuvant therapy except for the following: Prior systemic regimens must include a platinum-based therapy; investigational agents used in combination with standard therapies are allowed; tumors with driver mutations (epidermal growth factor receptor (EGFR) mutation positive or anaplastic lymphoma kinase fusion oncogene positive) treated with a tyrosine kinase inhibitor are permitted and are not considered a second systemic chemotherapy regimen (subjects should have progressed or be intolerant to the targeted therapy); maintenance or switch maintenance therapy after first-line chemotherapy is considered part of the 1 prior systemic chemotherapy regimen and is acceptable; subjects who completed and progressed on a platinum-containing regimen as adjuvant, neoadjuvant, or part of a course of chemoradiation therapy within the 6 months before screening are counted as having received 1 prior platinum-containing regimen and therefore do not require re-treatment with a platinum-containing regimen for Stage IIIB, IV, or recurrent disease; fresh baseline tumor biopsies, i.e., a biopsy specimen taken since completion of the most recent prior chemotherapy regimen, are required; and women of childbearing potential and males who use adequate birth control through 120 days after the last dose of study treatment).

The key exclusion criteria of the Phase 1/2 studies include the following: subjects who participated in any other study in which receipt of an investigational study drug or device occurred within 28 days or 5 half-lives (whichever is longer) before first dose. (For investigational agents with long half-lives (e.g., > 5 days), enrollment before the fifth half-life requires medical monitor approval); diagnosis of immunodeficiency or is receiving systemic steroid or any other form of immunosuppressive therapy within 7 days before the first dose of study treatment; prior monoclonal antibody within 4 weeks before study Day 1 or not recovered (≤ Grade 1 or at baseline) from adverse events (AEs) due to agents administered more than 4 weeks earlier.

For the Phase 2 study, the key exclusion criteria include the following: More than 1 prior systemic treatment for Stage IIIB, IV, or recurrent NSCLC; prior chemotherapy or targeted small molecule therapy within 2 weeks before study Day 1 or not recovered (≤ Grade 1 or at baseline) from AEs due to previously administered agents (Subjects with ≤ Grade 2 neuropathy are an exception and may enroll; if a subject received major surgery, he or she must have recovered adequately from the toxicity and/or complications from the intervention before starting therapy; prior therapy with an anti-PD-1, anti-PD-L1, anti-PD-L2, anti-CD137, or anti-cytotoxic T-lymphocyte-associated antigen-4 antibody (including ipilimumab or any other antibody or drug specifically targeting T-cell costimulation or checkpoint pathways); known additional malignancy that is progressing or requires active treatment (exceptions include basal cell carcinoma of the skin, squamous cell carcinoma of the skin, or *in situ* cervical cancer that has undergone potentially curative therapy); known active central nervous system (CNS) metastases and/or carcinomatous meningitis (Subjects with previously treated brain metastases may participate provided they are stable (without evidence of progression by imaging for at least 4 weeks before the first dose of study treatment and any neurologic symptoms have returned to baseline), have no evidence of new or enlarging brain metastases, and have not required steroids for at least 7 days before study treatment); active, or a documented history of, autoimmune disease, or a syndrome that has required systemic treatment in the past 2 years (i.e., with use of disease-modifying agents, corticosteroids, or immunosuppressive drugs). Replacement therapy (eg, thryoxine, insulin, or physiologic corticosteroid replacement therapy for adrenal or pituitary insufficiency, etc) is not considered a form of systemic treatment; evidence of interstitial lung disease or active, noninfectious pneumonitis; prior radiotherapy within 2 weeks of therapy (ubjects must have recovered from all radiation-related toxicities, not require corticosteroids, and not have had radiation pneumonitis; known hepatitis B virus (HBV) or hepatitis C virus (HCV) viremia or at risk for HBV reactivation (HBV DNA and HCV RNA must be undetectable. At risk for HBV reactivation is defined as: hepatitis B surface antigen positive or anti-hepatitis B core antibody positive); pregnant or nursing women or subjects expecting to conceive or father children within the projected duration of the study, starting with the screening visit through 120 days after the last dose of study treatment; known history of human immunodeficiency virus (HIV) (HIV 1/2 antibodies); live vaccine within 30 days before the first dose of study treatment; monoamine oxidase inhibitors within the 21 days before screening; any history of serotonin syndrome after receiving 1 or more serotonergic drugs; presence of a gastrointestinal condition that may affect drug absorption; history or current evidence of any condition, therapy, or laboratory abnormality that might confound the results of the study, interfere with the subject's participation for the full duration of the study, or is not in the best interest of the subject to participate, in the opinion of the treating investigator; known psychiatric or substance abuse disorders that would interfere with cooperation with the requirements of the study; immediate family member (self, spouse, or child) who is investigational site or sponsor staff directly involved with the study, unless prospective institutional review board approval (by chair or designee) is given allowing exception to this criterion for a specific subject; and known allergy or reaction to any component of either study drug formulation.

The Study Schedule and Procedures for the Phase 1/2 study are as follows: Subjects have regularly scheduled study visits at the clinical site on Day 1 of every cycle (CXD1) and midcycle in Cycle 1 (Day 8 ± 3 days), where laboratory assessments, vital sign collection, and physical examinations are performed. Liver function test monitoring occurs weekly during the first 6 weeks of study treatment during Phase 1 and then is tested every 3 weeks thereafter and in Phase 2 for subjects who remain on study treatment. Assessment of tumor size (by magnetic resonance imaging or computed tomography scan) is performed at screening or baseline (before beginning therapy), every 9 weeks for 18 months, and then every 12 weeks thereafter until disease progression. Disease progression is defined as progression confirmed by a second, consecutive assessment at least 4 weeks apart with the option for continuing treatment while awaiting radiologic confirmation of progression where feasible, and where subjects are clinically stable, defined as the following: absence of signs and symptoms (including worsening of laboratory values) indicating disease progression, no decline in ECOG performance status, absence of rapid progression of disease, and absence of progressive tumors at critical anatomical sites (e.g., cord compression) requiring urgent alternative medical intervention. All subjects are followed for survival at least every 12 weeks (± 7 days) until death.

Tumor biopsies are required at baseline and are optional any time after C1D14 or with confirmed response or progression in subjects with accessible tumors to assess tumor IDO1/PD-1/PD-L1 expression, degree and type of immune cell infiltration by immunohistochemistry, and other exploratory biomarkers, including mRNA expression profiling. Biopsy specimens obtained to evaluate toxicities are also collected to evaluate target-related expression. The standard PK parameters (e.g., t_{½} C_{SS}, Vd_{SS}, AUC, clearance) are determined for MK-3475 and INCB024360. All radiographic tumor assessments are collected for the option of independent review by a blinded, central reviewer. Central readings of images is not required before enrollment. All central reviews are retrospective and include review of the baseline scans and all on-study scans.

Primary endpoints of the Phase 1 study are as follows: Safety and tolerability are assessed by monitoring frequency, duration and severity of AEs, through physical examinations, by evaluating changes in vital signs and electrocardiograms, and through clinical laboratory blood and urine sample evaluations. Primary endpoints of the Phase 2 study are as follows: Progression-free survival, defined as the time from randomization until the earliest date of disease progression, as determined by investigator assessment of objective radiographic disease assessments per modified RECIST v1.1, or death due to any cause, if occurring sooner than progression. Imaging is archived for possible retrospective central confirmation of disease progression and response.

Phase 2 secondary endpoints include: objective response rate determined by radiographic disease assessments per modified RECIST (v1.1); ordinal categorical response score, determined by radiographic disease assessments per modified RECIST (v1.1); durability of response determined by radiographic disease assessment defined as the time from earliest date of disease response until earliest date of disease progression, and time to disease progression defined as the time from date of randomization until earliest date of disease progression; overall survival determined from the date of randomization until death due to any cause; and safety and tolerability of the treatment regimens through assessment of AEs and changes in safety assessments including laboratory parameters.

Exploratory endpoints in the study include: duration of disease control (including CR, PR, and SD) measuring from first report of SD or better until disease progression (Phase 2); objective response rate, PFS, and OS as defined above in PD-L1 high and low/negative subsets (Phase 2); summary of pharmacodynamics of INCB024360 and MK-3475 in whole blood and plasma (including plasma kynurenine/tryptophan ratio; peripheral blood immune cell population profile; and relevant plasma tumor markers and markers of immune modulation), (Phase 1 and Phase 2); tumor biopsy analyses to evaluate IDOl expression (+/-), PD-1/L1 expression (high/low-negative) in tumor tissue and immune cell infiltrate at baseline and correlate expression with response, PFS and/or OS; assessment of exploratory histological biomarkers (Phase 1 and Phase 2); summary of the PK of MK-3475 and INCB024360 as well evaluation of anti-MK-3475 antibodies. The PK of MK-3475 is compared between placebo and INCB024360 groups, while the PK of INCB024360 is assessed with concomitant MK-3475 administration (Phase 1 and Phase 2); assessment of the formation of antidrug antibodies to MK-3475 (Phase 1 and Phase 2); ORR, PFS and OS as defined above in subjects with advanced or metastatic disease enrolled in the Phase 1 portion of the study; progression-free survival and OS as defined above in subgroups of subjects with NSCLC, including subgroups based on prior tobacco use. (Phase 2).

An exploratory analysis is conducted for 5-category ordinal response at Week 24 determined by investigator and/or review using modified RECIST v1.1. The 5-category ordinal response endpoint is determined at a given timepoint by classifying a response into one of the following groups: CR; very good response, defined as PR with percent reduction from baseline in tumor line length > 60%; minor response, defined as PR with percent reduction from baseline in tumor line length ≤ 60%; SD; and PD.

For the Phase 1 study, descriptive statistics (e.g., mean, standard deviation, range) are derived where appropriate. Subject enrollment, disposition, demographics, and medical history are summarized at baseline. The rate of DLTs are summarized for each cohort. Dose exposure and density are calculated for each cohort. Safety and disease response data are compared over time to assess change from baseline, during treatment, and follow-up. Pharmacokinetic and PD data are analyzed with appropriate standard nonlinear analytic software. For the Phase 2 study, progression-free survival is analyzed by the Kaplan-Meier method after 52 PFS events are accrued in the randomized portion of the study. The hazard ratio (HR) and its 95% confidence interval are determined based on the Cox proportional hazards model using Efron's likelihood approximation to account for ties in event times. The sample size of 82 subjects (41 in each group, including 10% lost to follow-up) yields a power of 80% to detect a survival difference between INCB024360 in combination with MK-3475 and MK-3475 + placebo if the true HR is 0.5. This assumes a one-sided alpha of 0.05; an expected median PFS of 4 months in the MK-3475 + placebo group, 12-month enrollment, and 6 months of follow-up after the last subject has been randomized.

Table 3 below presents evaluable and confirmed preliminary efficacy data related to the clinical responses of subjects enrolled in the above-described Phase 1 study. More specifically, subjects were treated according to the Phase 1/2 study protocol with INCB024360 as shown in Table 3. In accordance with the protocol, INCB024360 was administered in combination with MK-3475. MK-3475 was intravenously administered at a dose of 2 mg/kg as described.

**TABLE 3**

| **Preliminary Clinical Efficacy Data from Ongoing Trial Patients, n (%)** | | |
|---|---|---|
| **Evaluable Response** | **INCB024360 25 mg BID (n = 4)** | **INCB024360 50 mg BID (n = 4)** |
| CR | 0 | 1 (33%) |
| PR | 2 (50%) | 2 (67%) |
| SD | 2 (50%) | 0 |
| PD | 0 | 0 |
| NE | 0 | 1** |

| | | |
|---|---|---|
| **NE: Not evaluable: One (1) subject died from a fall (unrelated to treatment) prior to the first on-study scans | | |

Table 3 presents preliminary efficacy data and results from subjects evaluated to date in the study described in this Example. In the 25 mg BID Cohort, a partial response (PR) was found in a subject having transitional cell carcinoma (TCC) in bladder (also called urothelial cell carcinoma or UCC). A PR was found in a second subject with melanoma (MEL). In addition, in the 25 mg BID Cohort, stable disease (SD) was found in a subject having melanoma (MEL); SD was also found in a second subject with non-small cell lung cancer (NSCLC).

For the evaluable subjects in the 50 mg BID Cohort, a complete response (CR) was found in a subject with melanoma (MEL). In the 50 mg BID cohort, a partial response (PR) was found in a subject with melanoma (MEL) and a PR was found in a second subject with renal carcinoma (renal cell cancer (RCC)). In the ongoing study, the 50 mg BID Cohort also has four enrolled subjects who are undergoing treatment, but who have not yet reached first on-study tumor assessment. Of these four study subjects receiving treatment, one has histologically or cytologically confirmed squamous cell carcinoma of the head and neck; one has adenocarcinoma of the endometrium; and two have renal cell cancer.

As described herein, subjects, patients, or individuals in the Phase 1 study are treated for different types of cancers. In particular aspects, a subject, patient, or individual undergoing treatment has a cancer selected from non-small-cell lung cancer (NSCLC), melanoma, transitional cell cancer of the bladder, renal cell cancer (RCC), triple negative breast cancer, adenocarcinoma of the endometrium, or squamous cell carcinoma of the head and neck.

Based on the preliminary data shown in Table 3, the total best overall response (per modified RECIST v1.1) was 71% (5/7), and the disease control rate was 100% (7/7), as calculated including only subjects evaluable for response (Objective response rate (ORR) 4/7 and Disease Control Rate (DCR) 7/7). As will be appreciated by the skilled practitioner, DCR as used herein refers to subjects, patients, or individuals who have stable disease or better (i.e., SD, PR, CR).

Table 4 below presents preliminary data of adverse events (AEs), including immune-related AEs, reported in the described study.

**TABLE 4**

| Type of AE | All Grades n (%) | | | Grade 3 or 4 n (%) | | |
|---|---|---|---|---|---|---|
| | 25 mg BID (n=4) | 50 mg BID (n=8) | Total (N=12) | 25 mg BID (n=4) | 50 mg BID (n=8) | Total (N=12) |
| Rash | 0 | 2 | 2 | 0 | 1 | 1 |
| Nausea | 2 | 1 | 3 | 0 | 0 | 0 |
| Cough | 2 | 0 | 2 | 0 | 0 | 0 |
| Diarrhea | 2 | 0 | 2 | | | |
| Dyspnea | 1 | 0 | 1 | 0 | 0 | 0 |
| Pruritis | 1 | 0 | 1 | 0 | 0 | 0 |
| Headache | 1 | 0 | 1 | 0 | 0 | 0 |
| DVT (IJ) | 1 | 0 | 1 | 0 | 0 | 0 |
| Fatigue | 0 | 1 | 1 | 0 | 0 | 0 |
| Mild Infusion Reaction | 0 | 1 | 1 | 0 | 0 | 0 |
| Fall | 0 | 1 | 1 | 0 | 1 | 1 |

In Table 4, one Grade 3 rash reported as an AE in the 50 mg BID cohort related to dose limiting toxicity (DLT) based on Extent of Rash. The subject recovered without steroids and continued on the study, with dose reductions, and has PR. One fall reported in the 50 mg BID cohort had a fatal outcome; however, the fall was not related to study treatment. Among the AEs listed in Table 4, DVT (IJ) refers to Deep Vein Thrombosis (Internal Jugular).

As observed in Table 3 above, the results, while preliminary, indicate promising anti-tumor activity of the combination therapy of the invention against multiple tumor types, and, in particular, against at least three different tumor types, including bladder, melanoma and renal tumors. In addition, the preliminary results indicate no major, study-related, adverse events or immune-related adverse events experienced by the subjects evaluated in the study.

Table 5 provides a brief description of the sequences in the Sequence Listing.

**TABLE 5**

| **SEQ ID NO:** | **Description** |
|---|---|
| **1** | hPD-1.08A light chain CDR1 |
| **2** | hPD-1.08A light chain CDR2 |
| **3** | hPD-1-08A light chain CDR3 |
| **4** | hPD-1.08A heavy chain CDR1 |
| **5** | hPD-1.08A heavy chain CDR2 |
| **6** | hPD-1.08A heavy chain CDR3 |
| **7** | hPD-1.09A light chain CDR1 |
| **8** | hPD-1.09A light chain CDR2 |
| **9** | hPD-1.09A light chain CDR3 |
| **10** | hPD-1.09A heavy chain CDR1 |
| **11** | hPD-1.09A heavy chain CDR2 |
| **12** | hPD-1.09A heavy chain CDR3 |
| **13** | 109A-H heavy chain variable region |
| **14** | 409A-H heavy chain full length |
| **15** | K09A-L-11 light chain variable region |
| **16** | K09A-L-16 light chain variable region |
| **17** | K09A-L-17 light chain variable region |
| **18** | K09A-L-11 light chain full length |
| **19** | K09A-L-16 light chain full length |
| **20** | K09A-L-17 light chain full length |
| **21** | MK-3475 Heavy chain |
| **22** | MK-3475 Light chain |
| **23** | Nivolumab Heavy chain |
| **24** | Nivolumab light chain |
| **25** | Human PD-L1 |

### REFERENCES

1. Sharpe, A.H, Wherry, E.J., Ahmed R., and Freeman G.J. The function of programmed cell death 1 and its ligands in regulating autoimmunity and infection. Nature Immunology (2007); 8:239-245.
2. Dong H et al. Tumor-associated B7-H1 promotes T-cell apoptosis: a potential mechanism of immune evasion. Nat Med. 2002 Aug; 8(8):793-800.
3. Yang et al. PD-1 interaction contributes to the functional suppression of T-cell responses to human uveal melanoma cells in vitro. Invest Ophthalmol Vis Sci. 2008 Jun; 49(6 (2008): 49: 2518-2525.
4. Ghebeh et al. The B7-H1 (PD-L1) T lymphocyte-inhibitory molecule is expressed in breast cancer patients with infiltrating ductal carcinoma: correlation with important high-risk prognostic factors. Neoplasia (2006) 8: 190-198.
5. Hamanishi J et al. Programmed cell death 1 ligand 1 and tumor-infiltrating CD8+ T lymphocytes are prognostic factors of human ovarian cancer. Proceeding of the National Academy of Sciences (2007): 104: 3360-3365.
6. Thompson RH et al. Significance of B7-H1 overexpression in kidney cancer. Clinical genitourin Cancer (2006): 5: 206-211.
7. Nomi, T. Sho, M., Akahori, T., et al. Clinical significance and therapeutic potential of the programmed death- 1 ligand/programmed death-1 pathway in human pancreatic cancer. Clinical Cancer Research (2007);13:2151-2157.
8. Ohigashi Y et al. Clinical significance of programmed death-1 ligand-1 and programmed death-1 ligand 2 expression in human esophageal cancer. Clin. Cancer Research (2005): 11: 2947-2953.
9. Inman et al. PD-L1 (B7-H1) expression by urothelial carcinoma of the bladder and BCG-induced granulomata: associations with localized stage progression. Cancer (2007): 109: 1499-1505.
10. Shimauchi T et al. Augmented expression of programmed death-1 in both neoplastic and nonneoplastic CD4+ T-cells in adult T-cell Leukemia/ Lymphoma. Int. J. Cancer (2007): 121:2585-2590.
11. Gao et al. Overexpression of PD-L1 significantly associates with tumor aggressiveness and postoperative recurrence in human hepatocellular carcinoma. Clinical Cancer Research (2009) 15: 971-979.
12. Nakanishi J. Overexpression of B7-H1 (PD-L1) significantly associates with tumor grade and postoperative prognosis in human urothelial cancers. Cancer Immunol Immunother. (2007) 56: 1173- 1182.
13. Hino et al. Tumor cell expression of programmed cell death-1 is a prognostic factor for malignant melanoma. Cancer (2010) 00:1-9.
14. Ghebeh H. Foxp3+ tregs and B7-H1+/PD-1+ T lymphocytes co-infiltrate the tumor tissues of high-risk breast cancer patients: implication for immunotherapy. BMC Cancer. (2008) 8:57.
15. Ahmadzadeh M et al. Tumor antigen-specific CD8 T cells infiltrating the tumor express high levels of PD-1 and are functionally impaired. Blood (2009) 114: 1537-1544.
16. Thompson RH et al. PD-1 is expressed by tumor infiltrating cells and is associated with poor outcome for patients with renal carcinoma. Clinical Cancer Research (2007) 15: 1757-1761.
17. Platten, M., W. Wick, and B.J. Van den Eynde, Tryptophan catabolism in cancer: beyond IDO and tryptophan depletion. Cancer Res (2012) 72(21): 5435-5440.
18. Munn, D.H., Blocking IDO activity to enhance anti-tumor immunity. Front Biosci (Elite Ed) (2012) 4:734-745.
19. Weber, W.P., et al., Differential effects of the tryptophan metabolite 3-hydroxyanthranilic acid on the proliferation of human CD8+ T cells induced by TCR triggering or homeostatic cytokines. Eur J Immunol (2006) 36(2):296-304.
20. Uyttenhove, C., et al., Evidence for a tumoral immune resistance mechanism based on tryptophan degradation by indoleamine 2,3-dioxygenase. Nat Med (2003) 9(10)1269-1274.
21. Liu, X., et al., Selective inhibition of IDO1 effectively regulates mediators of antitumor immunity. Blood (2010) 115(17):3520-3530.
22. Lee, G.K., et al., Tryptophan deprivation sensitizes activated T cells to apoptosis prior to cell division. Immunology (2002) 107(4)452-460.
23. Bonanno, G., et al., Indoleamine 2,3-dioxygenase 1 (IDO1) activity correlates with immune system abnormalities in multiple myeloma. J Transl Med (2012) 10:247.
24. Holmgaard, R.B., et al., Indoleamine 2,3-dioxygenase is a critical resistance mechanism in antitumor T cell immunotherapy targeting CTLA-4. J Exp Med (2013) 210 (7):1389-1402.
25. Yue, E.W., et al., Discovery of potent competitive inhibitors of indoleamine 2,3-dioxygenase with in vivo pharmacodynamic activity and efficacy in a mouse melanoma model. J Med Chem (2009) 52(23):7364-7367.
26. Koblish, H.K., et al., Hydroxyamidine inhibitors of indoleamine-2,3-dioxygenase potently suppress systemic tryptophan catabolism and the growth of IDO-expressing tumors. Mol Cancer Ther (2010) 9(2):489-498.
27. Liu, X., et al., Indoleamine 2,3-dioxygenase, an emerging target for anti-cancer therapy. Curr Cancer Drug Targets (2009) 9(8): p. 938-52.
28. Spranger, S.,et al., "Rational combinations of immunotherapeutics that target discrete pathways", Journal for ImmunoTherapy of Cancer, 2013, 1:16 : 1-14.

## Claims

1. A medicament comprising an antagonist of a Programmed Death 1 protein (PD-1) for use in combination with a IDOl inhibitor for treating a cancer in an individual, wherein the PD-1 antagonist is a monoclonal antibody with heavy chains each having the amino acid sequence of SEQ ID No: 21 and light chains each having the amino acid sequence of SEQ ID NO: 22; and the IDOl inhibitor is 4-({2-[(aminosulfonyl)amino]ethyl}amino)-N-(3-bromo-4-fluorophenyl)-N'-hydroxy-1,2,5-oxadiazole-3-carboximidamide or a pharmaceutically acceptable salt thereof.

2. The medicament for use according to claim 1, wherein the IDOl inhibitor is 4-({2-[(aminosulfonyl)amino]ethyl}amino)-N-(3-bromo-4-fluorophenyl)-N'-hydroxy-1,2,5-oxadiazole-3-carboximidamide.

3. The medicament for use according to claim 1 or claim 2, wherein the cancer is a solid tumor.

4. The medicament for use according to any one of claims 1 to 3, wherein the cancer is advanced renal cell carcinoma.

5. The medicament for use according to any one of claims 1 to 4, wherein the PD-1 antagonist is formulated as a liquid medicament which comprises 25 mg/ml anti-PD-1 monoclonal antibody, 7% (w/v) sucrose, 0.02% (w/v) polysorbate 80 in 10 mM histidine buffer pH 5.5.

6. A kit which comprises a first container, a second container and a package insert, wherein the first container comprises at least one dose of a medicament comprising an antagonist of a Programmed Death 1 protein (PD-1), the second container comprises at least one dose of a medicament comprising a IDOl inhibitor, and the package insert comprises instructions for treating an individual for cancer using the medicaments, and wherein the PD-1 antagonist is a monoclonal antibody with heavy chains each having the amino acid sequence of SEQ ID NO: 21 and light chains each having the amino acid sequence of SEQ ID NO: 22, and the IDOl inhibitor is 4-({2-[(aminosulfonyl)amino]ethyl}amino)-N-(3-bromo-4-fluorophenyl)-N'-hydroxy-1,2,5-oxadiazole-3-carboximidamide or a pharmaceutically acceptable salt thereof.

7. The medicament for use according to any one of claims 1, 2 or 5 or kit according to claim 6, wherein the cancer is bladder cancer, breast cancer, clear cell kidney cancer, head/neck squamous cell carcinoma, lung squamous cell carcinoma, melanoma, non-small-cell lung cancer (NSCLC), ovarian cancer, pancreatic cancer, prostate cancer, renal cell cancer, small-cell lung cancer (SCLC), triple negative breast cancer, endometrial cancer, acute lymphoblastic leukemia (ALL), acute myeloid leukemia (AML), chronic lymphocytic leukemia (CLL), chronic myeloid leukemia (CML), diffuse large B-cell lymphoma (DLBCL), follicular lymphoma, Hodgkin's lymphoma (HL), mantle cell lymphoma (MCL), multiple myeloma (MM), myeloid cell leukemia-1 protein (Mcl-1), myelodysplastic syndrome (MDS), non-Hodgkin's lymphoma (NHL), or small lymphocytic lymphoma (SLL).

8. The medicament for use according to any one of claims 1 to 5, wherein the PD-1 antagonist is administered to the individual in an amount of 2 mg/kg and the IDOl inhibitor is administered to the subject at a dose of 25 mg or 50 mg.

9. The medicament for use according to any one of claims 1 to 5, wherein the PD-1 antagonist is administered to the individual in an amount of 200 mg and the IDO1 inhibitor is administered to the subject at a dose of 25 mg or 50 mg.

10. The medicament for use according to claim 8 or claim 9, wherein the IDOl inhibitor is administered to the individual at a dose of 25 mg BID, or wherein the IDOl inhibitor is administered to the individual at a dose of 50 mg BID.

11. The medicament for use according to claim 8 or claim 9, wherein the PD-1 antagonist is administered every three weeks and one dose of the IDOl inhibitor is administered two times per day, preferably wherein the IDOl inhibitor is administered at twelve hour intervals.

12. The medicament for use according to any one of claims 8 to 11, wherein the PD-1 antagonist and the IDOl inhibitor are dosed over a 21-day dosing period.

13. The medicament for use according to any one of claims 8 to 12, wherein the cancer is non-small-cell lung cancer (NSCLC), melanoma, transitional cell cancer of the bladder (TCC), renal cell cancer (RCC), or squamous cell carcinoma of the head and neck.

14. The medicament for use according to any one of claims 1 to 5 wherein
a) the PD-1 antagonist is administered to the subject in an amount of 10 mg/kg one dose every two weeks or one dose every three weeks and the IDOl inhibitor is administered to the subject at a dose of 25 mg BID, 50 mg BID, 100 mg BID, or 300 mg BID; or
b) the PD-1 antagonist is administered to the subject in an amount of 2 mg/kg one dose every two weeks or one dose every three weeks and the IDOl inhibitor is administered to the subject at a dose of 25mg BID.

## Patentansprüche

1. Medikament, umfassend einen Antagonisten eines Programmed Death-1-Proteins (PD-1)zur Verwendung in Kombination mit einem IDO1-Inhibitor zur Behandlung einer Krebserkrankung in einem Individuum, wobei der PD-1-Antagonist ein monoklonaler Antikörper ist, dessen schwere Ketten jeweils die Aminosäuresequenz von SEQ ID NO: 21 aufweisen, und die leichten Ketten jeweils die Aminosäuresequenz von SEQ ID NO: 22 aufweisen, und der IDO1-Inhibitor 4-({2-[(Aminosulfonyl)amino]ethyl}-amino)-N-(3-brom-4-fluorphenyl)-N'-hydroxy-1,2,5-oxadiazol-3-carboximidamid oder ein pharmazeutisch annehmbares Salz davon ist.

2. Medikament zur Verwendung nach Anspruch 1, wobei der IDO1-Inhibitor 4-({2-[(Aminosulfonyl)amino]ethyl}-amino)-N-(3-brom-4-fluorphenyl)-N'-hydroxy-1,2,5-oxadiazol-3-carboximidamid ist.

3. Medikament zur Verwendung nach Anspruch 1 oder Anspruch 2, wobei es sich bei der Krebserkrankung um einen soliden Tumor handelt.

4. Medikament zur Verwendung nach einem der Ansprüche 1 bis 3, wobei es sich bei der Krebserkrankung um fortgeschrittenes Nierenzellkarzinom handelt.

5. Medikament zur Verwendung nach einem der Ansprüche 1 bis 4, wobei der PD-1-Antagonist als flüssiges Medikament formuliert ist, das 25 mg/ml monoklonalen anti-PD-1-Antikörper, 7% (Gew./Vol.) Saccharose, 0,02% (Gew./Vol.) Polysorbat 80 in 10 mM Histidinpuffer pH 5,5 umfasst.

6. Kit, das einen ersten Behälter, einen zweiten Behälter und eine Packungsbeilage umfasst, wobei der erste Behälter mindestens eine Dosis eines Medikaments umfasst, das einen Antagonisten eines Programmed Death-1-Proteins (PD-1) umfasst, der zweite Behälter mindestens eine Dosis eines Medikaments umfasst, umfassend einen IDO1-Inhibitor, und die Packungsbeilage Anweisungen zur Behandlung eines Individuums gegen Krebs unter Verwendung der Medikamente enthält, und wobei der PD-1-Antagonist ein monoklonaler Antikörper ist, dessen schwere Ketten jeweils die Aminosäuresequenz von SEQ ID NO: 21 aufweisen, und die leichten Ketten jeweils die Aminosäuresequenz von SEQ ID NO: 22 aufweisen, und der IDO1-Inhibitor 4-({2-[(Aminosulfonyl)amino]ethyl}amino)-N-(3-brom-4-fluorphenyl)-N'-hydroxy-1,2,5-oxadiazol-3-carboximidamid oder ein pharmazeutisch annehmbares Salz davon ist.

7. Medikament zur Verwendung nach einem der Ansprüche 1, 2 oder 5 oder Kit nach Anspruch 6, wobei die Krebserkrankung Blasenkrebs, Brustkrebs, klarzelliger Nierenkrebs, Kopf-/Hals-Plattenepithelkarzinom, Lungen-Plattenepithelkarzinom, Melanom, nicht-kleinzelliger Lungenkrebs (NSCLC), Eierstockkrebs, Bauchspeicheldrüsenkrebs, Prostatakrebs, Nierenzellkrebs, kleinzelliger Lungenkrebs (SCLC), dreifach negativer Brustkrebs, Endometriumkrebs, akute lymphatische Leukämie (ALL), akute myeloische Leukämie (AML), chronische lymphatische Leukämie (CLL), chronische myeloische Leukämie (CML), diffuses großes B-Zell-Lymphom (DLBCL), follikuläres Lymphom, Hodgkin-Lymphom (HL), Mantelzell-Lymphom (MCL), multiples Myelom (MM), Myeloidzell-Leukämie-1-Protein (Mcl-1), myelodysplastisches Syndrom (MDS), Non-Hodgkin-Lymphom (NHL) oder kleines lymphozytisches Lymphom (SLL).

8. Medikament zur Verwendung nach einem der Ansprüche 1 bis 5, wobei der PD-1-Antagonist dem Individuum in einer Menge von 2 mg/kg verabreicht wird und der IDO1-Inhibitor dem Individuum in einer Dosis von 25 mg oder 50 mg verabreicht wird.

9. Medikament zur Verwendung nach einem der Ansprüche 1 bis 5, wobei der PD-1-Antagonist dem Individuum in einer Menge von 200 mg verabreicht wird und der IDO1-Inhibitor dem Individuum in einer Dosis von 25 mg oder 50 mg verabreicht wird.

10. Medikament zur Verwendung nach Anspruch 8 oder Anspruch 9, wobei der IDO1-Inhibitor dem Individuum in einer Dosis von 25 mg zweimal täglich verabreicht wird, oder wobei der IDO1-Inhibitor dem Individuum in einer Dosis von 50 mg zweimal täglich verabreicht wird.

11. Medikament zur Verwendung nach Anspruch 8 oder Anspruch 9, wobei der PD-1-Antagonist alle drei Wochen verabreicht wird, und eine Dosis des IDO1-Inhibitors zweimal täglich verabreicht wird, wobei der IDO1-Inhibitor vorzugsweise in Intervallen von zwölf Stunden verabreicht wird.

12. Medikament zur Verwendung nach einem der Ansprüche 8 bis 11, wobei der PD-1-Antagonist und der IDO1-Inhibitor über einen Dosierungszeitraum von 21 Tagen dosiert werden.

13. Medikament zur Verwendung nach einem der Ansprüche 8 bis 12, wobei die Krebserkrankung nicht-kleinzelliger Lungenkrebs (NSCLC), Melanom, Transitionalzellkarzinom der Harnblase (TCC), Nierenzellkrebs (RCC) oder Plattenepithelkarzinom des Kopfes und des Halses ist.

14. Medikament zur Verwendung nach einem der Ansprüche 1 bis 5, wobei
a) der PD-1-Antagonist dem Individuum in einer Menge von 10 mg/kg in einer Dosis alle zwei Wochen oder in einer Dosis alle drei Wochen verabreicht wird und der IDO1-Inhibitor dem Individuum in einer Dosis von 25 mg zweimal täglich, 50 mg zweimal täglich, 100 mg zweimal täglich oder 300 mg zweimal täglich verabreicht wird; oder
b) der PD-1-Antagonist dem Individuum in einer Menge von 2 mg/kg in einer Dosis alle zwei Wochen oder in einer Dosis alle drei Wochen verabreicht wird und der IDO1-Inhibitor dem Individuum in einer Dosis von 25 mg zweimal täglich verabreicht wird.

## Revendications

1. Médicament comprenant un antagoniste de la protéine Programmed Death 1 (PD-1) pour une utilisation en combinaison avec un inhibiteur d'IDO1 pour le traitement d'un cancer chez un individu, l'antagoniste de PD-1 étant un anticorps monoclonal possédant des chaînes lourdes chacune possédant la séquence d'acides aminés de SEQ ID No : 21 et des chaînes légères chacune possédant la séquence d'acides aminés de SEQ ID No : 22 ; et l'inhibiteur d'IDO1 étant le 4-({2-[(aminosulfonyl)amino]éthyl}amino)-N-(3-bromo-4-fluorophényl)-N'-hydroxy-1,2,5-oxadiazole-3-carboximidamide ou un sel pharmaceutiquement acceptable correspondant.

2. Médicament pour une utilisation selon la revendication 1, l'inhibiteur d'IDO1 étant le 4-({2-[(aminosulfonyl)amino]éthyl}amino)-N-(3-bromo-4-fluorophényl)-N'-hydroxy-1,2,5-oxadiazole-3-carboximidamide.

3. Médicament pour une utilisation selon la revendication 1 ou la revendication 2, le cancer étant une tumeur solide.

4. Médicament pour une utilisation selon l'une quelconque des revendications 1 à 3, le cancer étant un carcinome des cellules rénales avancé.

5. Médicament pour une utilisation selon l'une quelconque des revendications 1 à 4, l'antagoniste de PD-1 étant formulé comme un médicament liquide qui comprend 25 mg/ml d'anticorps monoclonal anti-PD-1, 7 % (p/v) de saccharose, 0,02 % (p/v) de polysorbate 80 dans un tampon d'histidine 10 mM à pH 5,5.

6. Kit qui comprend un premier récipient, un deuxième récipient et un insert d'emballage, le premier récipient comprenant au moins une dose d'un médicament comprenant un antagoniste d'une protéine Programmed Death 1 (PD-1), le deuxième récipient comprenant au moins une dose d'un médicament comprenant un inhibiteur d'IDO1, et l'insert d'emballage comprenant des instructions pour le traitement d'un cancer chez un individu à l'aide des médicaments, et l'antagoniste de PD-1 étant un anticorps monoclonal possédant des chaînes lourdes chacune possédant la séquence d'acides aminés de SEQ ID No : 21 et des chaînes légères chacune possédant la séquence d'acides aminés de SEQ ID No : 22, et l'inhibiteur d'IDO1 étant le 4-({2-[(aminosulfonyl)amino]éthyl}amino)-N-(3-bromo-4-fluorophényl)-N'-hydroxy-1,2,5-oxadiazole-3-carboximidamide ou un sel pharmaceutiquement acceptable correspondant.

7. Médicament pour une utilisation selon l'une quelconque des revendications 1, 2 et 5 ou kit selon la revendication 6, le cancer étant un cancer de la vessie, un cancer du sein, un cancer du rein à cellules claires, un carcinome à cellules squameuses de la tête/du cou, un carcinome à cellules squameuses du poumon, un mélanome, un cancer du poumon non à petites cellules (NSCLC), un cancer de l'ovaire, un cancer pancréatique, un cancer de la prostate, un cancer de cellules rénales, un cancer du poumon à petites cellules (SCLC), un cancer du sein triplement négatif, un cancer endométrial, une leucémie lymphoblastique aiguë (ALL), une leucémie myéloïde aiguë (AML), une leucémie lymphocytaire chronique (CLL), une leucémie myéloïde chronique (CML), un lymphome à grande cellules B diffus (DLBCL), un lymphome folliculaire, un lymphome de Hodgkin (HL), un lymphome à cellules du manteau (MCL), un myélome multiple (MM), une protéine de leucémie-1 de cellules myéloïdes (Mcl-1), un syndrome myélodysplasique (MDS), un lymphome non de Hodgkin (NHL), ou un petit lymphome lymphocytaire (SLL).

8. Médicament pour une utilisation selon l'une quelconque des revendications 1 à 5, l'antagoniste de PD-1 étant administré à l'individu en une quantité de 2 mg/kg et l'inhibiteur d' IDO1 étant administré au sujet à raison d'une dose de 25 mg ou 50 mg.

9. Médicament pour une utilisation selon l'une quelconque des revendications 1 à 5, l'antagoniste de PD-1 étant administré à l'individu en une quantité de 200 mg et l'inhibiteur d'IDO1 étant administré au sujet à raison d'une dose de 25 mg ou 50 mg.

10. Médicament pour une utilisation selon la revendication 8 ou la revendication 9, l'inhibiteur d'IDO1 étant administré à l'individu à raison d'une dose de 25 mg BID, ou l'inhibiteur d'IDO1 étant administré à l'individu à raison d'une dose de 50 mg BID.

11. Médicament pour une utilisation selon la revendication 8 ou la revendication 9, l'antagoniste de PD-1 étant administré toutes les trois semaines et une dose de l'inhibiteur d'IDO1 étant administrée deux fois par jour, préférablement l'inhibiteur d'IDO1 étant administré à des intervalles de douze heures.

12. Médicament pour une utilisation selon l'une quelconque des revendications 8 à 11, l'antagoniste de PD-1 et l'inhibiteur d'IDO1 étant dosés sur une période de dosage de 21 jours.

13. Médicament pour une utilisation selon l'une quelconque des revendications 8 à 12, le cancer étant un cancer du poumon non à petites cellules (NSCLC), un mélanome, un cancer à cellules transitionnelles de la vessie (TCC), un cancer de cellules rénales (RCC), ou un carcinome à cellules squameuses de la tête et du cou.

14. Médicament pour une utilisation selon l'une quelconque des revendications 1 à 5,
a) l'antagoniste de PD-1 étant administré au sujet en une quantité d'une dose de 10 mg/kg toutes les deux semaines ou d'une dose toutes les trois semaines et l'inhibiteur d'IDO1 étant administré au sujet à raison d'une dose de 25 mg BID, 50 mg BID, 100 mg BID, ou 300 mg BID ; ou
b) l'antagoniste de PD-1 étant administré au sujet en une quantité d'une dose de 2 mg/kg toutes les deux semaines ou d'une dose toutes les trois semaines et l'inhibiteur d'IDO1 étant administré au sujet à raison d'une dose de 25 mg BID.
